# EUROPEAN PATENT APPLICATION

(11) **EP 2 568 050 A2**
(43) Date of publication of application: **13.03.2013**
(21) Application number: 12194773.3
(22) Date of filing: 01.08.2008
(51) Int. Cl.: C12Q 1/04, C12Q 1/24

(54) **Pathogen Detection in Large-Volume Particulate Samples**

(30) Priority: 01.08.2007 US 953422 P; 01.11.2007 US 984624 P; 31.12.2007 US 18079 P; 21.05.2008 US 55099 P
(62) Divisional of application: 08797058.8
(71) Applicant: Hitachi Chemical Co., Ltd., Shinjuku-ku, Tokyo 163-0449 (JP); HITACHI CHEMICAL RESEARCH CENTER, INC., Irvine, CA 92617 (US)
(72) Inventor: Murakami, Taku, Aliso Viejo, CA California 92656 (US); Li, Jifan, Irvine, CA California 92614 (US); Mitsuhashi, Masato, Irvine, CA California 92614 (US); Oakes, Melanie, San Juan Capristano, CA California 92675 (US)
(74) Representative: Watson, Robert James

(57) **Abstract**

Methods and filter systems for detecting microorganisms in a sample, such as a food sample, are disclosed. The filter is configured to attract the microorganism, for example by electrostatic charge. The filter containing the microorganism is incubated to grow the microorganism so that it may be detected. The filter may be degradable and the detection may involve extracting the microorganism or the molecular marker of the microorganism from the filter for detection. The filter system may also include a porous microbead component selected to trap dirt and other contaminants from the sample while allowing the microorganisms to pass among the microbeads. Methods are disclosed for detecting microorganisms in samples using the filter systems described. The methods may also include adding a polymer and/or a microorganism detection reagent to the filter to localize microorganism growth and prevent evaporation of reagents.

## Description

### Related Applications

This application claims priority to U.S. Provisional Application No. 60/953,422, filed August 1, 2007, U.S. Provisional Application No. 60/984,624, filed November 1, 2007, U.S. Provisional Application No. 61/018,079, filed December 31, 2007, and U.S. Provisional Application No. 61/055,099, filed May 21, 2008. All of the above-referenced applications are incorporated herein by reference.

### Field of the Invention

The present disclosure relates to a reagent, method, and apparatus for detecting pathogens in large volume particulate samples.

### BACKGROUND

### Description of the Related Art

According to a recent estimate by the Centers for Disease Control (CDC), food-borne pathogens are the cause of 76 million cases of food-borne illness, 325,000 hospitalizations, and 5,000 deaths within the United States alone each year. In addition to the harmful medical effects on humans, these food-borne outbreaks have detrimental economic effects due to medical costs, lost productivity, product recalls, and halted exports. The USDA recommends a zero-tolerance policy for certain food-borne pathogens, as even a small amount of pathogen can survive and cause food poisoning or even an outbreak. Therefore, the development of sensitive pathogen detection technology capable of detecting even a single pathogen in food samples is necessary.

Many detection technologies and products are currently available for the detection of food-borne pathogens, but it is still challenging to detect a single pathogen contaminating a few grams of food sample within a 24 hour time frame. Generally, pathogens are extracted from a food sample by dilution and homogenization, resulting in a sample volume of as much as a few hundred mL, or even larger. In order to detect small numbers of pathogens in a large volume of sample, it is necessary to culture the cells until the pathogen concentration reaches a level suitable for a pathogen detection assay. The necessary pre-enrichment time depends on the doubling time, the viability of the target pathogen, and the pathogen concentration required for detection. Pre-enrichment usually takes at least 12 to 48 hours. Therefore, even using highly sensitive detection assays such as polymerase chain reaction (PCR) and enzyme-linked immunosorbent assay (ELISA), the detection of pathogen contamination in a food sample still requires one to two days. Additionally, these assays are generally expensive and require skilled laboratory personnel.

Furthermore, food pathogen assays with cultural pre-enrichment require handling enriched pathogen, which may cause secondary contamination of laboratories and personnel. Thus, these assays are not appropriate for on-site testing at food manufacturing plants and may instead need to be conducted off-site at, for example, reference laboratories. Also, cultural pre-enrichment will cause the loss of quantitative information regarding contaminated food pathogens, because the viability of contaminated pathogens and the ingredients of food samples can affect pathogen growth rate and because how quickly samples reach the stationary phase or the decline phase of cell growth may depend on the pathogen contamination level. Therefore, food pathogen assays with pre-enrichment are non-quantitative and estimating the level of food pathogen contamination is difficult.

In order to overcome the aforementioned disadvantages of pathogen assays with pre-enrichment, alternative enrichment procedures such as centrifugation or filtration of a large sample volume to increase the pathogen concentration may be necessary. However, centrifugation requires a centrifuge and is generally a cumbersome process, especially when a large number of large volume samples need to be tested.

Filtration may be performed in higher throughput and can concentrate even a low number of pathogens present in large volume samples. To concentrate pathogens, a filtration method generally utilizes microfiltration (MF) membrane filters, which generally have pore sizes smaller than the target pathogen. Thus, pathogens can be concentrated on such filters and detected by several detection methods, such as colony formation and DNA probe hybridization. However, these membrane filters have very low dirt-holding capacities and tend to readily get clogged with particulate food extracts due to their small pore sizes. These filters are acceptable for samples containing less particulate, such as drinking or environmental water, or for very small volumes of particulate samples. Depth filters with an appropriate retention rate are also used to trap pathogens; however, the detection of pathogens trapped in such depth filters is complicated by their three-dimensional matrix structure and filter thickness.

### SUMMARY

Embodiments of the invention are directed to methods of detecting a microorganism in a sample by one or more of the following steps:
filtering the sample through a filter configured to attract the microorganism and having pores configured to prevent clogging during filtration, such that the microorganism is collected in the filter,
applying a polymer and a microorganism detection reagent to the filter; the polymer is configured to localize growth of the microorganism and/or prevent evaporation of the microorganism detection reagent;
incubating the filter for a period of time sufficient to grow the microorganism to detectable level; and
detecting the presence of the microorganism in the sample.

In preferred embodiments, the polymer is a hydrogel. Preferably, the hydrogel is an environment sensitive hydrogel. The environment sensitive hydrogel may be at least partially in sol form with a low viscosity before application of an environmental change and becomes at least partially gel form with increased viscosity upon application of the environmental change. In preferred embodiments, the environmental change is temperature, pH, amount of incident light, ion concentration, pressure, magnetic field, electric field, sonic radiation, or biochemical molecule concentration. In preferred embodiments, the application of the environment sensitive hydrogel to the filter includes steps of applying a solution having at least partially sol form of the environment sensitive hydrogel to the filter; and gelling the solution by application of the environmental change.

In preferred embodiments, the hydrogel is a hydrogel particle. Preferably, the hydrogel particle is applied by a method such as absorption, aspiration, filtration, or soak and spray of a suspension of the hydrogel particle. In preferred embodiments, the hydrogel particle is smaller than the filter pores, thereby occupying the filter pores efficiently.

In preferred embodiments, the hydrogel is applied to the filter pores in dehydrated form, and then hydrated inside the filter pores. Preferably, hydration of the dehydrated hydrogel takes a period of time from 1 minute to 6 hours. Preferably, the dehydrated hydrogel is applied to the filter pores in a solution, and is hydrated inside the pores by incubation for 1 minute to 6 hours.

In preferred embodiments, the polymer applied to the filter is a viscous polymer solution. The viscous polymer solution flows into the filter, thereby filling the pores and localizing the growth of the microorganisms effectively. Preferably, the viscous polymer solution includes a water-soluble polymer or hydrogel suspension.

In preferred embodiments, the microorganism detection reagent applied to the filter contains a growth medium configured to selectively promote the growth of the microorganism. Preferably, the growth medium includes a selective reagent or antibiotic to which the microorganism is resistant but other organisms are not resistant, a selected pH and temperature for fast growth of the microorganism and/or slow or inhibited growth of other organisms, or a nutrient readily metabolized by the microorganism but not readily metabolized by other organisms. In some preferred embodiments, the growth medium includes sufficient oxygen for the growth of aerobic bacteria or insufficient oxygen for the growth of anaerobic bacteria.

In preferred embodiments, the detecting step includes detecting a signal from a signaling reagent configured to generate the signal in the presence of the microorganism, and thereby detecting the microorganism. Preferably, the signaling reagent is a substrate for an enzyme specific to the microorganism, a chromogenic or fluorogenic substrate for an enzyme specific to the microorganism, or a combination of a substrate for an enzyme specific to the microorganism and a chromogenic or fluorogenic indicator to detect digestion of the substrate.

In some preferred embodiments, the signaling reagent is an agent recognizing the microorganism or a cellular component of the microorganism, such as an antibody, an antigen, an atpamer, a protein, a nucleic acid or a carbohydrate. Preferably, the agent has a label such as from a dye molecule, a metal colloid, a polymer particle, a magnetic particle, a semiconductive particle, a liposome, a polymersome, a protein, an enzyme or a nucleic acid.

Alternate embodiments of the invention are directed to methods which include separation of the microorganism from a particulate sample by filtering the particulate sample with a layered filter composite having a filter configured to attract a microorganism and having pores configured to prevent clogging during filtration; and also including a layer of porous spherical microbeads. Preferably, the method includes detecting the microorganism immobilized in the filter.

Embodiments of the invention may also include washing the filter after the filtering step in the methods described above for a period of time sufficient to remove substances that inhibit the detection or growth of the microorganism.

In preferred embodiments, the filter in the described methods is a depth filter having a fibrous material. Preferably, the fibrous material is cellulose fibers or glass microfibers.

Preferably, the filter is configured to attract the microorganism by an interaction such as electrostatic, hydrophilic, hydrophobic, physical, or biological interactions. In preferred embodiments, an agent recognizing the microorganism is immobilized specifically or non-specifically on the filter matrix. The agent may be an antibody, an antigen, an aptamer, a protein, a nucleic acid, or a carbohydrate.

In preferred embodiments, the filter in the methods described above further includes a layer of porous spherical microbeads. In some preferred embodiments, the layer of porous spherical microbeads is placed on the upstream surface of the filter. In some preferred embodiments, the layer of porous spherical microbeads is placed on the upstream surface of a mesh support. The mesh support is placed on the filter or upstream of the filter.

In preferred embodiments, the layer of porous spherical microbeads forms a homogeneous or graded layer. Preferably, the surface of the layer of porous spherical microbeads is highly inert and has low non-specific binding to the microorganism.

In preferred embodiments, the diameter of the microbeads is between 1 and 1000 µm, more preferably the diameter of the microbeads is between 15 and 600 µm, and yet more preferably the diameter of the microbeads is between 50 and 300 µm. Preferably, the pore size of the microbeads is smaller than the size of the microorganism and the microorganism passes among the microbeads during the filtration step. In some preferred embodiments, the microbeads include a cross-linked hydrophilic polymer.

In some preferred embodiments, the layer of porous spherical microbeads is disrupted by adding a wash medium after the filtration step, followed by suspending the microbeads; and filtering the wash medium with the filter.

Embodiments of the invention are directed to kits for detecting a microorganism which include one or more of the following components:
a filter configured to attract the microorganism and having pores configured to prevent clogging during filtration;
a microorganism detection reagent configured to detect the presence of the microorganism; and
a polymer configured to localize growth of the microorganism and/or prevent evaporation of the microorganism detection reagent.

In preferred embodiments, the filter is a depth filter having a fibrous material. Preferably, the fibrous material is selected from cellulose fibers and glass microfibers.

Preferably, the filter is configured to attract the microorganism by an interaction such as electrostatic, hydrophilic, hydrophobic, physical, and biological interactions.

In preferred embodiments, the detection reagent recognizing the microorganism is immobilized specifically or non-specifically on the filter matrix. The agent is preferably an antibody, an antigen, an aptamer, a protein, a nucleic acid, or a carbohydrate.

In preferred embodiments, the polymer component of the kit is a hydrogel. Preferably, the hydrogel is an environment sensitive hydrogel, which is at least partially in sol form. The environment sensitive hydrogel has low viscosity before application of an environmental change and becomes at least partially gel form with increased viscosity upon application of the environmental change. Preferably, the environmental change is a change in temperature, pH, amount of incident light, ion concentration, pressure, magnetic field, electric field, sonic radiation, or biochemical molecule concentration.

In preferred embodiments, the hydrogel is a hydrogel particle. Preferably, the hydrogel particle is smaller than the filter pores, thereby occupying the filter pores efficiently. More preferably, the hydrogel may be in dehydrated form.

In preferred embodiments, the filter in the kit also includes a layer of porous spherical microbeads. In some preferred embodiments, the layer of porous spherical microbeads is placed on the upstream surface of the filter. In some preferred embodiments, the layer of porous spherical microbeads is placed on the upstream surface of a mesh support and the mesh support is placed on the filter or upstream of the filter.

In preferred embodiments, the layer of porous spherical microbeads forms a homogeneous or graded layer. In preferred embodiments, the surface of the layer of porous spherical microbeads is highly inert and has low non-specific binding to the microorganism.

In preferred embodiments, the diameter of the microbeads is between 1 and 1000 µm, more preferably the diameter of the microbeads is between 15 and 600 µm and yet more preferably the diameter of the microbeads is between 50 and 300 µm.

In preferred embodiments, the pore size of the microbeads is smaller than the size of the microorganism, whereby the microorganism passes among the microbeads during the filtration step. In some preferred embodiments, the microbeads include a cross-linked hydrophilic polymer.

In preferred embodiments, the microorganism detection reagent in the kit contains a growth medium configured to selectively promote the growth of the microorganism. Preferably, the growth medium includes a selective reagent or antibiotic to which the microorganism is resistant but other organisms are not resistant, a selected pH and temperature for fast growth of the microorganism and/or slow or inhibited growth of other organisms, or a nutrient readily metabolized by the microorganism but not readily metabolized by other organisms. In some preferred embodiments, the growth medium includes sufficient oxygen for the growth of aerobic bacteria or insufficient oxygen for the growth of anaerobic bacteria.

In preferred embodiments, the growth medium includes a signaling reagent configured to generate the signal in the presence of the microorganism, and thereby detecting the microorganism. Preferably, the signaling reagent is an enzyme specific to the microorganism, a chromogenic or fluorogenic substrate for an enzyme specific to the microorganism, or a combination of a substrate for an enzyme specific to the microorganism and an chromogenic or fluorogenic indicator to detect digestion of the substrate.

In some preferred embodiments, the signaling reagent is an agent recognizing the microorganism or a cellular component of the microorganism, such as an antibody, an antigen, an atpamer, a protein, a nucleic acid or a carbohydrate, and the agent preferably includes a label such as a dye molecule, a metal colloid, a polymer particle, a magnetic particle, a semiconductive particle, a liposome, a polymersome, a protein, an enzyme and a nucleic acid.

Embodiments of the invention are directed to a layered filter composite for detecting a microorganism including a filter medium configured to attract a microorganism and having pores configured to prevent clogging during filtration; and a layer of porous spherical microbeads.

In preferred embodiments, the filter medium in the layered filter composite is a depth filter having a fibrous material. Preferably, the fibrous material is cellulose fibers or glass microfibers.

In preferred embodiments, the filter medium of the layered filter composite is configured to attract microorganisms by an interaction such as electrostatic, hydrophilic, hydrophobic, physical, and biological interactions. In preferred embodiments, an agent recognizing the microorganism is immobilized specifically or non-specifically on the filter matrix, and the agent is selected from an antibody, an antigen, an aptamer, a protein, a nucleic acid, and a carbohydrate.

In preferred embodiments, the layer of porous spherical microbeads in the layered filter composite is placed on the upstream surface of the filter. In other preferred embodiments, the layer of porous spherical microbeads is placed on the upstream surface of a mesh support and the mesh support is placed on the filter or upstream of the filter. Preferably, the layer of porous spherical microbeads forms a homogenous or graded layer. Preferably, the surface of the layer of porous spherical microbeads is highly inert and has low non-specific binding to the microorganism.

In preferred embodiments, the diameter of the microbeads is between 1 and 1000 µm, more preferably the diameter of the microbeads is between 15 and 600 µm, and yet more preferably the diameter of the microbeads is between 50 and 300 µm. In preferred embodiments, the pore size of the microbeads is smaller than the size of the microorganism, whereby the microorganism passes among the microbeads during the filtration step. In some embodiments, the microbeads include a cross-linked hydrophilic polymer.

Embodiments of the invention are directed to a porous filter, which includes a hydrogel which is a three-dimensional polymer network that can swell, but does not dissolve in water; and a filter configured to attract a microorganism and having pores configured to prevent clogging during filtration.

In preferred embodiments, the hydrogel of the porous filter is in dehydrated form.

In preferred embodiments, the filter has a filter matrix which includes fibrous materials.

In preferred embodiments, the three-dimensional polymer network of the hydrogel includes a polymer such as agar, chitosan, cellulose, carrageenan, alginate, acrylate polymer and copolymer, polyethylene glycol, poly(glycolic acid), poly(lactic acid), pectin, locust bean gum, polyhydroxybutyrate (PHB), polyhydroxyvalerate (PHV), polycaprolactone, polydioxanone, polyanhydrides, polycyanoacrylates, poly(amino acids), poly(ortho ester), polyphosphazenes, poly(propylene fumarate), poly(alkylene oxalates), silicone polymers, collagen, fibrinogen, fibrin, gelatin, starch, amylose, dextran, silk, keratin, elastin, actin, myosin, glycosaminoglycans, or polyvinyl alcohol.

In preferred embodiments, the filter is configured to attract the microorganism by an interaction such as electrostatic, hydrophilic, hydrophobic, physical, and biological interactions.

In preferred embodiments, an agent recognizing the microorganism is immobilized specifically or non-specifically on the filter matrix. Preferably, the agent is selected from a metal hydroxide or metal oxide, an antibody, an antigen, an aptamer, a protein, a nucleic acid, or a carbohydrate. Preferably, the metal hydroxide or metal oxide is zirconium hydroxide, titanium hydroxide, hafnium oxide, hydroxyapatite, iron oxide, titanium oxide, or aluminum oxide.

In preferred embodiments, the filter contains a microorganism detection reagent, and retains the microorganism detection reagent during filtration of a microorganism containing sample. Preferably, the microorganism detection reagent includes a growth medium configured to selectively promote the growth of the microorganism to be detected and a signaling reagent configured to generate a signal in the presence of the microorganism. Preferably, the growth medium includes a selective reagent or antibiotic to which the microorganism is resistant but other organisms are not resistant, a selected pH and temperature for fast growth of the microorganism and/or slow or inhibited growth of other organisms, or a nutrient readily metabolized by the microorganism but not readily metabolized by other organisms. In some preferred embodiments, the growth medium has sufficient oxygen for the growth of aerobic bacteria or insufficient oxygen for the growth of anaerobic bacteria.

In preferred embodiments, the signaling reagent is selected from a substrate for an enzyme specific to the microorganism, a chromogenic or fluorogenic substrate for an enzyme specific to the microorganism, and a combination of a substrate for an enzyme specific to the microorganism and a chromogenic or fluorogenic indicator to detect digestion of the substrate.

In preferred embodiments, the signaling reagent is an agent recognizing the microorganism or a cellular component of the microorganism, such as an antibody, an antigen, an atpamer, a protein, a nucleic acid and a carbohydrate. Preferably, the agent includes a label such as a dye molecule, a metal colloid, a polymer particle, a magnetic particle, a semiconductive particle, a liposome, a polymersome, a protein, an enzyme or a nucleic acid.

Embodiments of the invention are directed to the use of the porous filter described above in a method of detecting a microorganism in a sample with one or more of the following steps:
immobilizing a microorganism by filtration of the sample through the porous filter;
incubating the filter for a period of time sufficient to grow the microorganism to detectable levels; and
detecting the microorganism using a signaling reagent.

In some preferred embodiments, the porous filter is soluble after filtration. For example, the porous filter may include a degradable polymer. Preferably, the degradable polymer is provided in a form such as fiber, surface coating, beads, particles, sheet or layer. In some preferred embodiments, the degradable polymer becomes a solution via hydrolysis, oxidative, or enzymatic mechanism.

In some embodiments, the degradable porous filter is used to detect a microorganism in a sample by one or more of the following steps:
(a) immobilizing a microorganism by filtration of the sample through the filter;
(b) degrading the degradable filter to become a solution;
(c) extracting the microorganism or a molecular marker specific to the microorganism from the solution; and
(d) detecting the microorganism or the microorganism specific molecular marker, thereby confirming the presence of the microorganism in the sample.

Further aspects, features and advantages of this invention will become apparent from the detailed description of the preferred embodiments which follow.

### BRIEF DESCRIPTION OF THE DRAWINGS

These and other feature of this invention will now be described with reference to the drawings of preferred embodiments which are intended to illustrate and not to limit the invention.

Figure 1 shows a schematic depiction of porous spherical beads assisted filtration.

Figure 2 shows a schematic depiction of the steps of the pathogen detection assay procedure using an environment-sensitive hydrogel.

Figure 3 shows a schematic depiction of the steps of the pathogen detection assay procedure using hydrogel microbeads.

Figures 4A through 4D show various configurations of hydrogel composite filter. Figure 4A shows an embodiment in which the hydrogel is positioned between filter matrix layers. Figure 4B shows an embodiment in which hydrogel particles are positioned between filter matrix layers. Figure 4C shows an embodiment in which hydrogel particles are dispersed throughout the filter matrix. Figure 4D shows an embodiment in which hydrogel fibers form the filter matrix.

Figure 5 shows a composite hydrogel filter comprising smart hydrogel particles with a pathogen detection reagent incorporated therein.

Figures 6A through 6C show various configurations of degradable filter. Figure 6A shows an embodiment in which the filter membrane is made of degradable particles. Figure 6B shows an embodiment in which the filter membrane is made of degradable fibers. Figure 6C shows an embodiment in which the filter membrane is made of water soluble particles with degradable coating.

Figure 7 shows diagrammatically an assay using degradable filter to collect and detect the pathogen.

Figure 8 shows the results of pathogen detection assays for 250 mL 10% whole milk samples filtered with glass fiber depth filters. Figures 8A and 8B show the results for milk samples inoculated with 200 cfu *Listeria* cells after 40-hour incubation at 30°C. Figures 8C and 8D show the results for milk samples with no *Listeria* cells after 40-hour incubation at 30°C.

Figure 9 shows a microscopic image of hydrogel microparticles. Microparticles were stained with trypan blue for higher contrast.

Figure 10A through E shows the results of pathogen detection assays for 235, 23.5, 2.35, 0.235, and 0 cfu *Listeria* cells per 10 mL BHI broth filtered with electropositive filters.

Figure 11 shows the filtration speed of 10% deli meat (A) and 10% stick cheese homogenates (B) using an electropositive depth filter with 2 g porous microbeads (●), an electropositive depth filter with 15 g non-porous glass beads (■), and an electropositive depth filter without a filter aid (A).

Figure 12 shows the filtration speed of 10% deli meat (A) and 10% frankfurter homogenates (B) using a glass fiber depth filter with several grades of porous spherical microbeads filter aids: Cross-linked polymethacrylate microbeads (45 - 90 µm diameter: 0.5 g (○), 1.0 g (●)) and cross-linked dextran microbeads (17 - 70 µm diameter: 0.5 g (□), 35 - 140 µm diameter: 0.5 g (0), 86 - 258 µm diameter: 0.5 g (Δ), 1.0 g (A), 170 - 520 µm diameter: 0.5 g (×)).

Figure 13 shows the results of pathogen detection assays for 220 cfu (Figures 13A, 13B), 22 cfu (Figures 13C, 13D), and 0 cfu (Figures 13E, 13F) *Listeriα* cells per ∼225 mL 10% deli meat homogenate (25 g deli meat) filtered with glass fiber depth filters and porous microbeads filter aids. Figures 13A, 13C, and 13E show the results after 24-hour incubation and Figures 13B, 13D, and 13F show the results after 40-hour incubation at 30°C.

Figure 14 shows the results of pathogen detection assays in ∼225 mL 10% deli meat homogenates (25 g deli meat) filtered with glass fiber depth filters and porous microbeads filter aids. A. The numbers of colored spots observed from the upstream (top) and downstream sides (bottom) of the filters are summarized in the table. B. The total numbers of observed colored spots were plotted against the inoculated pathogen concentration. Closed and open circle indicate *Listeria* monocytogenes and *Listeria* innocua, respectively.

Figure 15 shows the result of an assay on a fabricated filter showing *Listeria* cells that are found on the filter after incubation.

Figure 16 shows the result of an assay on a composite filter with pathogen detection reagents immobilized by a smart hydrogel.

Figure 17 shows the PCR results of an assay on a degradable filter for pathogen detection. Cycle indicates the number of PCR cycles and ΔR_{N} indicates normalized relative fluorescent intensity in each reaction after subtraction of the baseline intensity.

### DETAILED DESCRIPTION OF THE PREFERRED EMBODIMENTS

While the described embodiment represents the preferred embodiment of the present invention, it is to be understood that modifications will occur to those skilled in the art without departing from the spirit of the invention. The scope of the invention is therefore to be determined solely by the appended claims.

The present disclosure relates to a method that allows sensitive detection of pathogens in a large volume of particulate sample with low cost and a short hands-on time, to the fabrication of filters employed in said method, and to the use of a porous spherical microbeads filter aid in said method.

### Pathogen Detection Filtration Assay Using Highly Porous Filter, Porous Spherical Microbeads Filter Aid and Hydrogel Materials

In one embodiment, the disclosed method utilizes a highly porous filter with pathogen adsorption capability and high dirt holding capacity in order to efficiently concentrate a pathogen from a large volume particulate sample. Highly porous filters useful in embodiments of the disclosed method may attract target pathogens by electrostatic, hydrophilic, hydrophobic, physical, or biological interactions and also may be porous enough to prevent filter clogging by particulate samples. In certain embodiments, the filter deployed is a depth filter with a three-dimensional matrix that provides high dirt holding capacity and the ability to trap pathogens. The useful depth filter may be made of fibrous materials such as glass fiber and nitrocellulose fiber, and comprise of a single or multilayer filter with the same or different particle retention rate. The filter has appropriate particle retention rate and thickness to trap pathogens efficiently and to avoid filter clogging due to particles in the sample. For example, in order to trap a pathogen such as *Listeria,* which is 0.5 - 2 µm in length and 0.4 - 0.5 µm in diameter, in a depth filter, appropriate particle retention rate of depth filters may be 0.1 to 10 µm, preferably 0.7 to 2.4 µm. Also, the filter has sufficient mechanical strength to withstand vacuum forces or pressure applied during filtration.

In other embodiments, the filter is a depth filter that uses electropositive charges to attract pathogens, as pathogen surfaces usually have a net negative charge on account of the lipopolysaccharides, teichoic acids, and surface proteins contained therein. The pathogen may be immobilized by the electropositive charges on the filter rather than by the filter matrix, thus making it possible to further increase filter porosity and obtain a higher dirt-holding capacity to avoid filter clogging more efficiently. The electropositive charges may be provided by surface coating of filter matrix with cationic molecules or incorporating electropositive colloids, particles or fibers made of electropositive materials. The examples of the electropositive materials are metal hydroxides and metal oxides, such as zirconium hydroxide, titanium hydroxide, hafnium oxide, iron oxide, titanium oxide, aluminum oxide, and hydroxyapatite. Preferably, the isoelectric point of the metal hydroxides or metal oxides may be higher than the pH values of the sample and detection reagent. There are a few rare instances of positively-charged bacteria, such as *Stenotrophomonas maltophilia.* Such microorganisms can be more readily retained by filters with an electronegative charge, which can be prepared in a manner similar to the electropositive filters described above, using electronegative charges rather than electropositive ones.

In other embodiments, the filter comprises pathogen recognition agents such as antibodies, antigens, proteins, nucleic acids, carbohydrates, aptamers, or bacteriophages. These pathogen recognition agents can recognize pathogens selectively or non-selectively vis-à-vis other microorganisms found in the sample and can be immobilized on the filter matrix by chemical binding, physical binding, or other standard immobilization methods. In certain embodiments, the pathogen recognition agent is a toll-like receptor (TLR), which recognizes structurally conserved molecules present on the surface of various microorganisms. TLR2 can recognize Gram-positive peptidoglycan and lipoteichoic acid, and TLR4 can recognize lipopolysaccharides on Gram-negative bacteria.

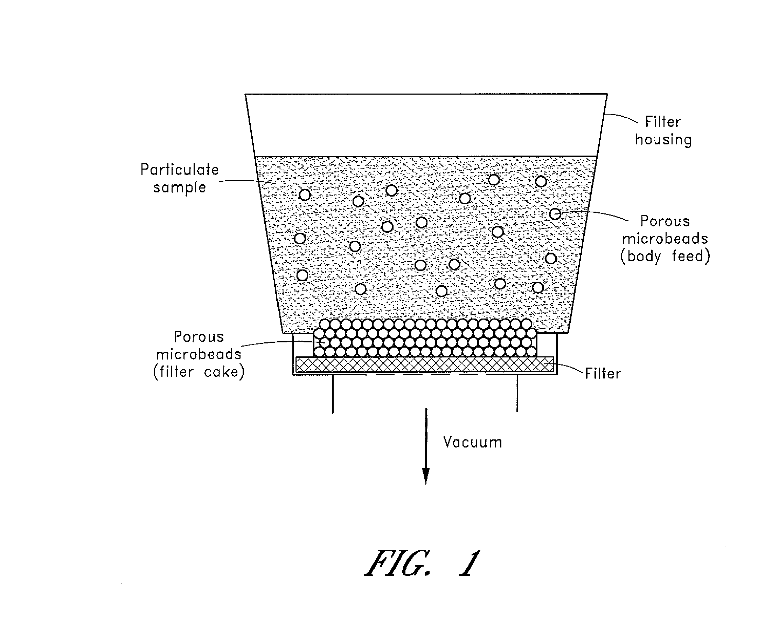

In addition to the filter, the disclosure relates to the use of a porous spherical microbeads filter aid to prevent filter clogging by particulates in the sample to be tested (Figure 1). Useful microbeads may be spherically, spheroidally or ellipsoidally shaped porous microbeads with a small size distribution. The microbeads may take the closest packed structure such as cubic closest packed structure and hexagonal closest packed structure or close structure to that. The microbeads may typically have a diameter of 1 to 1000 µm, preferably 15 to 600 µm, and more preferably 50 to 300 µm, in order for the pathogen to be tested to pass among the microbeads during sample filtration. The useful microbeads have appropriate specific gravity to be suspended but not to float in water, buffer, growth medium or sample solution. The microbeads may typically have pores that are smaller than a target pathogen, thereby preventing the pathogen from being trapped inside the pores of the porous spherical microbeads during sample filtration. Preferably, the microbeads may have an inert surface, preferably hydrophilic surface, and have low non-specific binding to biomolecules and microorganisms, including proteins, nucleic acids, carbohydrates, bacteria, viruses, and cells. In certain embodiment, the microbeads filter aid is made of cross-linked polymer such as polymethacrylate and dextran. Optionally, the microbeads can remove materials that may inhibit the downstream detection reaction on account of their porous structure.

A porous spherical microbeads filter aid may be used to aid filtration of a large volume particulate sample at least in one of the following ways or a combination thereof. A filter aid can be placed as a homogeneous or graded layer of porous spherical microbeads on the upstream surface of a highly porous filter with pathogen adsorption capability before sample filtration. Alternatively, a filter aid can be added to the particulate sample before filtration and form a layer of porous spherical microbeads during sample filtration, thereby providing new layers of the microbeads continuously and further improving filtration. A mesh support may be placed between the highly porous filter and the filter aid layer in order to remove the filter aid layer easily after sample filtration. Optionally, the porous microbeads filter aid is pre-incubated or suspended in a solution containing a blocking reagent such as a peptide or protein before use in order to minimize pathogen binding to the microbeads surface.

The pathogen to be detected may pass among the microbeads and may not be trapped on the surfaces or in the pores of the microbeads. On the other hand, particles in the sample, that typically clog a filter without a filter aid layer, may be trapped on the microbeads surface and in the gaps among the porous spherical microbeads. Because of the porous structure of the microbeads, the sample streams during filtration not only pass among the beads but also penetrate the microbeads themselves, therefore the sample particles tend to be trapped on the beads surface than in the gaps among the beads, and thereby the porous spherical filter aid layer can provide high dirt holding capacity and prevent filter clogging during filtration of large volume particulate samples. After complete filtration of the sample, the filter aid layer can be disrupted and the microbeads can be suspended in a wash solution, and the wash solution can be filtered to collect the pathogen that may be trapped in the filter aid layer during the initial filtration of the particulate sample, thereby improving pathogen immobilization yield in the filter. This wash process can be repeated several times to maximize the pathogen recovery yield in the filter. The wash solution is typically a buffer solution or growth medium that is not harmful to the pathogen.

In addition to the filter and the filter aid, the disclosure relates to the use of a polymer material to prevent the evaporation of a pathogen detection reagent during a pathogen detection reaction and to localize the growth of the pathogen in the filter. Useful polymer materials include natural or synthetic polymers such as polysaccharide and protein. Some examples of the polymer materials include, but are not limited to, agar, alginate, carrageenan, cellulose, chitosan, dextran, pectin, collagen, fibrinogen, acrylate polymer, polyethylene glycol, polyvinyl alcohol including their derivatives and copolymers. The polymer material may be preferred to form a hydrogel by intra- and/or inter-molecular cross-linking. A hydrogel is a three-dimensional polymer network that can swell but that does not dissolve in water, and that can retain water or solution without evaporation for a certain period of time.

In certain embodiments, the hydrogel is an environment-sensitive hydrogel. Useful environment-sensitive hydrogels may respond to one or more environmental stimuli such as temperature, pH, light, ions, pressure, magnetic field, electric field, sonic radiation, or biochemical molecules. When an environment-sensitive hydrogel is in sol form, the material is in the form of a water-soluble polymer, however, as a result of the application of the stimulus, the material converts to gel form (hydrogel) and becomes water-insoluble and cross-linked intra- or inter-molecularly. The conversion of sol and gel forms may occur completely or partially. In certain embodiments, the environment-sensitive hydrogel and environmental stimulation is not harmful to pathogens, especially when live pathogens are to be detected. In certain embodiments, the sol form is not too viscous to flow easily into the filter pores in order to fill the pores easily, and the gel form prevents the evaporation of pathogen detection reagents and localizes the growth of the pathogen effectively. Some examples of the environment-sensitive hydrogel includes, but is not limited to, agarose, low melting point agarose, carrageenan, pectin, methylcellulose and Pluronic F127. Agarose is in sol form at ∼45°C and gels upon cooling to ∼37°C. Low-melting-point agarose is in sol form at ∼37°C and gels at ∼20°C. Carrageenan and pectin are in sol form in the absence of certain ions such as potassium and calcium and gels on addition of these ions. Methylcellulose and Pluronic F127 are in sol form at ∼4°C and gel after warming to ∼37°C. In certain embodiments, the appropriate hydrogel concentration may vary for different hydrogels. In certain embodiments, the appropriate concentration of agarose or low melting point agarose is 0.01% to 30%, preferably 0.1% to 10%, and more preferably 0.5% to 5%. In certain embodiments, the sol form can be applied to the depth filter pores after sample filtration by absorption, aspiration, capillary action, filtration, immersion, spray or other techniques, and converted to gel form by environmental stimulation.

In certain embodiments, the filter pores are filled with hydrogel particles after sample filtration. The hydrogel particles may be monodisperse or polydisperse. Useful geometries of hydrogel particles include, but are not limited to, spherical, tetrahedral, hexahedral, polyhedral, or columnar. However, any regular or irregular shape can be used. In order to fill the filter pores effectively with hydrogel particles, the hydrogel particles may be smaller than the filter pores in order to go into the filter pores. Optionally, the hydrogel particles may be large enough to be retained in the filter and may be larger than the retention rate of the highly porous filter. Application of hydrogel particles into the filter pores can be done by absorption, aspiration, capillary action, filtration, immersion, spray or other techniques. Useful hydrogel particles can be synthesized or prepared by several conventional techniques such as molding, emulsion polymerization/gellation, precipitation polymerization, membrane emulsification, cutting, slicing and grinding. In some embodiments, the hydrogel particles are made of agarose gel and the appropriate agarose concentration is 0.01% to 10% (w/v), and is more preferably 0.1% to 5% (w/v).

In certain embodiments, the filter pores are filled with hydration action of dehydrated hydrogel. When dehydrated hydrogel swells upon hydration, hydrogel volume increases dramatically. Dehydrated hydrogel can be applied to the filter pores by absorption, aspiration, capillary action, filtration, immersion, spray or other techniques, and hydration of the dehydrated hydrogel can be done after application of a solution such as water, buffer or growth medium. In certain embodiments, the dehydrated hydrogel may be monodisperse or polydisperse particles and the size of the dehydrated hydrogel particles may be small enough to effectively enter the filter pores. Dehydrated hydrogel particles can be prepared by dehydration of hydrogel particles through heating, drying, freeze-drying and any other techniques. In certain embodiments, hydration action of the dehydrated hydrogel particles takes a certain period of time ranging 10 seconds to 6 hours, more preferably 1 to 30 minutes, thereby dehydrated hydrogel particles suspended in a solution can be applied to the filter pores by the above mentioned techniques before hydration and then hydrated inside the pores through incubation.

In certain embodiments, the filter pores are filled with a viscous polymer solution, which may be not too viscous to flow easily into the filter pores in order to fill the pores easily, but viscous enough to localize the growth of the pathogen effectively. The polymer solution may include a water-soluble polymer or a hydrogel suspension.

In certain embodiments, the pathogen detection reagent contains a growth medium for a pathogen. The growth medium generally includes one or more of a carbon source, nitrogen source, amino acids, and various salts for pathogen growth. In certain embodiments, the growth medium may be a selective medium, which selectively promotes the growth of a specific pathogen by an antibacterial/antifungal effect, temperature or pH resistance, or the ability to synthesize a certain metabolite, thereby allowing the pathogen to grow selectively vis-à-vis other competing bacteria or microorganisms. In some embodiments, the pathogen detection reagent may contain compounds such as L-cysteine and Oxyrase (Oxyrase Inc., Mansfield, OH) to accelerate the growth of specific pathogens or resuscitation of injured pathogens by reducing oxygen concentration in the growth medium.

In some embodiments, the pathogen detection reagent may contain a signaling reagent that differentiates a pathogen from other bacteria or microorganisms. One example of said signaling reagent is a chromogenic or fluorogenic substrate for a pathogen-specific enzyme. Examples of said substrates include: X-, Lapis-, Magenta-, Salmon-, Green-, ONP- and MU-linked substrates such as beta-D-glucuronide, beta-D-galacto-pyranoside, beta-D-gluco-pyranoside, N-acety-beta-D-galactosaminide, N-acetyl-beta-D-glucosaminide, caprylate-nonanoate, butyrate, myo-inositol-1-phosphate, and phosphate. Another example of said signaling reagent is the combination of a pH indicator such as phenol red and a substrate for a pathogen-specific enzyme such as xylose. Metabolization of the substrate changes pH of the detection reagent, and the pH indicator indicates pH changes in a colorimetric manner. Other examples of said signaling reagents are a nucleic acid including DNA, RNA, LNA, PNA or other nucleic acid, an antibody, an antigen or an aptamer to recognize the pathogen or pathogen specific cellular components. Those probe molecules may be labeled by a fluorescent dye molecule, a gold or silver colloid, a polymer particle, a magnetic particle, a semiconductive particle, a quantum dot, a photonic crystal particle, a liposome, a polymersome, an enzyme, a protein and a nucleic acid for signal generation.

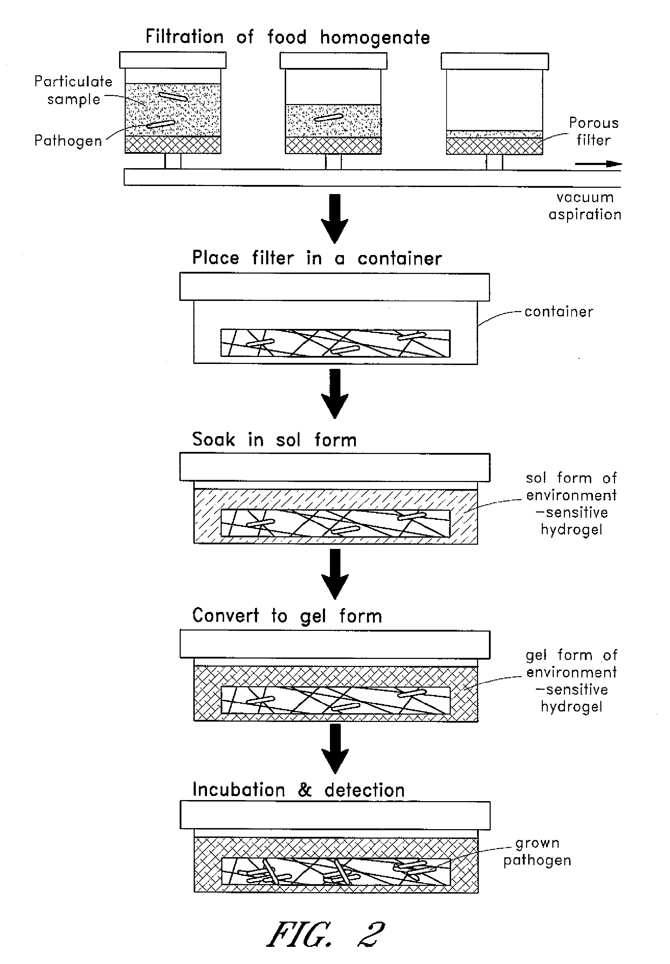

In certain embodiments, pathogen detection in a large volume of particulate sample using an environment-sensitive hydrogel comprises the following steps (Figure 2):
(1) Filter a large volume of particulate sample that may contain the pathogen to be detected through a layered filter comprising a highly porous filter and a porous microbeads filter aid, thereby collecting the target pathogen in the filter.
(2) If necessary, wash the filter and the porous microbeads filter aid in order to maximize recovery of the pathogen in the filter and remove any potential inhibitors of a pathogen detection assay.
(3) Collect the filter and apply sol form of an environment-sensitive hydrogel containing a pathogen detection reagent to fill the filter pores by absorption, immersion, spray, or other techniques.
(4) Apply an environmental stimulus to convert the sol form of the environment-sensitive hydrogel to gel form.
(5) Incubate the hydrogel-containing filter for a period of time sufficient for pathogen growth such that the pathogen will be detectable.
(6) Detect the grown pathogen using said signaling reagent.

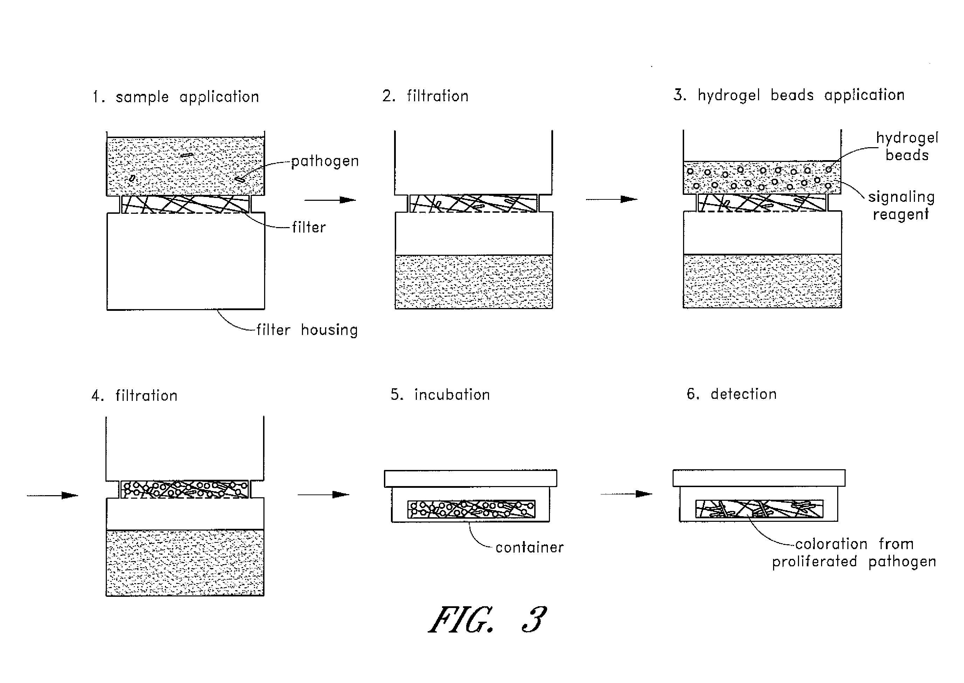

In certain embodiments, pathogen detection in a large volume of particulate sample using hydrogel particles comprises the following steps (Figure 3):
(1) Filter a large volume of particulate sample that may contain the pathogen to be detected through a layered filter comprising a highly porous filter and a porous microbeads filter aid, thereby collecting a target pathogen in the filter.
(2) If necessary, wash the filter and/or the porous microbeads filter aid in order to maximize recovery of the pathogen in the filter and remove any potential inhibitors of a pathogen detection assay.
(3) Collect the filter and apply hydrogel particles containing a pathogen detection reagent to fill the filter pores.
(4) Incubate the hydrogel-particle-containing filter for a period of time sufficient for pathogen growth such that the pathogen will be detectable.
(5) Detect the grown pathogen using said signaling reagent.

Optionally, in order to resuscitate and detect an injured pathogen, the particulate sample may be prepared and incubated in a growth medium or solution allowing resuscitation of the injured pathogen before sample filtration and pathogen detection. Alternatively, an injured pathogen can be resuscitated in the filter after sample filtration through incubation, which may be done in a growth medium, solution or environment allowing resuscitation of the injured pathogen, and detected by a pathogen detection reagent. Alternatively, the pathogen detection reagent is configured to resuscitate an injured pathogen, and the concentration, component or pH of the pathogen detection reagent may be changed during incubation by supplementing additional reagents such as antibiotics with or without the hydrogel materials described herein, thereby the injured pathogen can be resuscitated and detected.

Filtration of the particulate sample and application of a hydrogel-containing detection reagent may require manual handling, and these processes may be completed within one hour. These processes can be readily automated using standard automation instrumentation. The incubation time required for pathogen growth and detection depends on the doubling time of the pathogen and the sensitivity of the signaling reagent. In order to detect a single pathogen in the filter, the required incubation time may be as shown in Table 1. Even when a less sensitive chromogenic substrate is used to identify the pathogen, the total assay may be completed within 24 hours with a very short hands-on time.

**Table 1. Required Incubation Time to Detect a Single Pathogen**

| **Pathogen doubling time** | **Detection limit of signaling reagents** | | | | |
|---|---|---|---|---|---|
| | **10 cfu** | **10² cfu** | **10³ cfu** | **10⁴ cfu** | **10⁵ cfu** |
| **20 min.** | 1.1 hrs | 2.2 hrs | 3.3 hrs | 4.4 hrs | 5.5 hrs |
| **60 min.** | 3.3 hrs | 6.6 hrs | 10.0 hrs | 13.3 hrs | 16.6 hrs |

In certain embodiments, alternative pathogen detection assays can be used after pathogen immobilization in the highly porous filter. One useful pathogen detection method is filter incubation in a selective culture medium with a pathogen-specific enzyme substrate without hydrogel. A chromogenic or fluorogenic enzyme substrate, or a combination of a pH indicator such as phenol red and a substrate for a pathogen-specific enzyme such as xylose, may be used to detect the pathogen. The optical density, optical absorption, or fluorescent intensity of the culture medium can be measured or monitored after or throughout the incubation in order to detect the presence of the pathogen, or the grown pathogen in the culture medium can be detected by a conventional pathogen detection assays such as polymerase chain reaction (PCR), reverse transcription polymerase chain reaction (RT-PCR), nucleic acid sequence based amplification (NASBA), loop-mediated isothermal amplification (LAMP), any other isothermal nucleic acid amplification, enzyme linked immunosorbent assay (ELISA), immunochromatography, biosensor, or nucleic acid probe. Alternatively, the pathogen or cellular components of the pathogen such as genomic DNA, ribosomal RNA, transfer RNA, messenger RNA, or protein, which indicate the presence of the specific pathogen, can be extracted from the filter by use of a detergent, chaotropic reagent, organic solvent, or electrophoresis with or without breaking the filter structure, and detected using the conventional pathogen detection methods. Alternatively, a pathogen immobilized in a filter can be detected by applying a sensing agent to the filter. A useful sensing agent may be immobilized inside the filter by electrostatic, hydrophilic, hydrophobic, physical, or biological interactions. An example of a sensing agent is a pathogen-specific antibody immobilized B-cell as disclosed in U.S. Patent No. 6,087,114 and Rider et al.*,* "A B Cell-Based Sensor for Rapid Identification of Pathogens," Science, 301, 213-15 (2003).

Examples of pathogens that may be detected include pathogenic bacteria and other microorganisms infectious or harmful to humans, animals, plants, the environment, and/or industry. Examples of pathogenic bacteria include, but are not limited to, *Escherichia, Salmonella, Listeriα, Campylobacter, Shigella, Brucella, Helicobactor, Mycobacterium, Streptococcus,* and *Pseudomonas.* Pathogenic virus can be detected in combination with a conventional pathogen detection method as disclosed herein. Examples of pathogenic virus families include, but are not limited to, *Adenoviridae, Picornaviridae, Herpesviridae, Hepadnaviridae, Flaviviridae, Retroviridae, Orthomyxoviridae, Paramyxoviridae, Papovaviridae, Rhabdoviridae,* and *Togaviridae.* In addition, the disclosed filtration system is useful for detecting both pathogenic and non-pathogenic microorganisms in large volumes of particulate samples.

Examples of particulate samples that can be tested include, but are not limited to, food samples, homogenates of food samples, wash solutions of food samples, drinking water, ocean/river water, environment water, mud and soil. Additionally, swabs, sponges and towels wiping a variety of environment surfaces can be tested as well. In addition, the disclosed filtration system is useful to test other large volume particulate samples including human body fluids, urine, blood and manufacturing water/solution. Samples, especially solid forms, can be diluted, homogenized and/or filtered with a mesh having 50 - 1000 µm pores before sample filtration for more efficient filtration.

### Composite Hydrogel Filter for Pathogen Detection

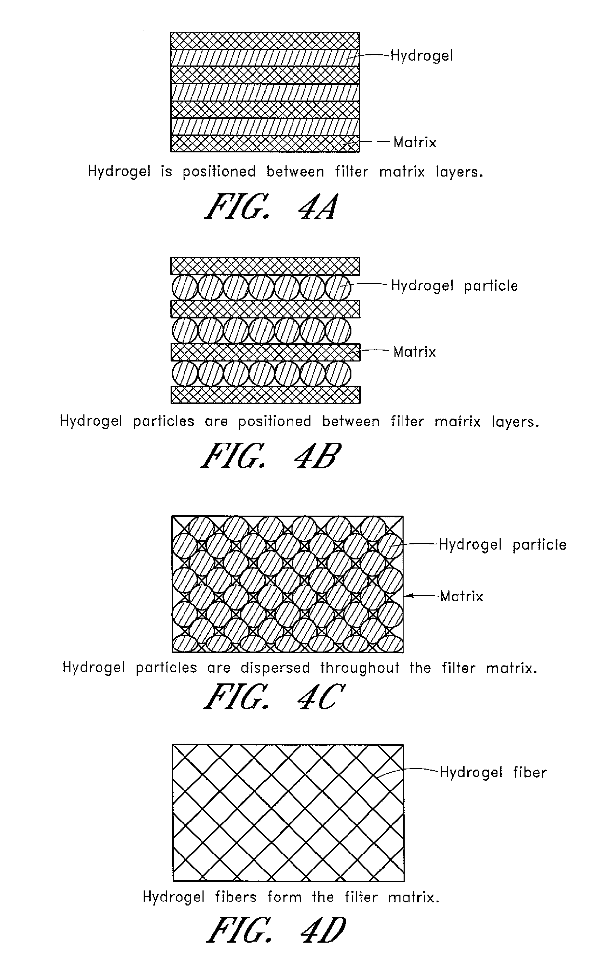

In certain embodiments, a hydrogel is included in the highly porous filter with pathogen recognition capability to form a composite hydrogel filter. The composite hydrogel filter can be fabricated in many ways. In one embodiment, the hydrogel or hydrogel particles can be placed on the top, bottom, or inside of the filter matrix, which comprises fibrous materials such as glass fiber or nitrocellulose fiber. In another embodiment, the filter matrix is formed from the hydrogel fibers. Since hydrogels are three-dimensional polymer networks, they are highly porous in their dehydrated state. The porosity can be adjusted depending on the polymerization method used to generate the hydrogel. In one embodiment, a sample solution can penetrate through pores inside the hydrogel during filtration. In another embodiment, the solution can be filtered through the gaps between hydrogel particles. Thus the composite hydrogel filter cannot be blocked during filtration. Various embodiments of the composite hydrogel filter are illustrated in Figures 4A-4D.

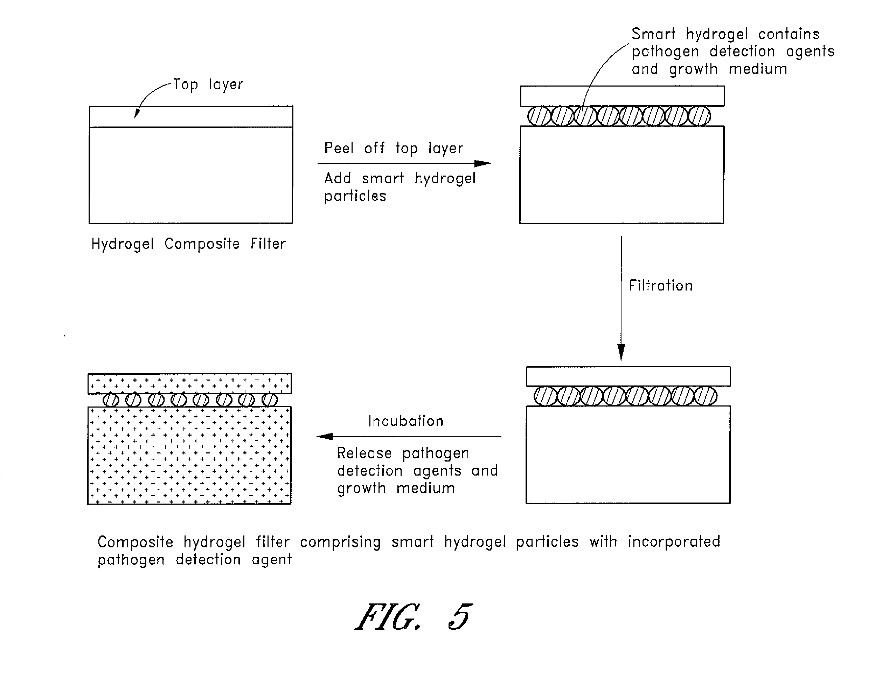

In one embodiment, the composite hydrogel filter contains a pathogen detection reagent inside or on the surface of the hydrogel and retains the reagent efficiently even after filtration of the sample solution to be tested. In another embodiment, an environment-sensitive hydrogel (smart hydrogel) is used to hold and release a pathogen detection reagent in order to prevent elution of the pathogen detection reagent during sample filtration. The smart hydrogel can respond to small physical, biological, or chemical stimuli with large property changes such as shrinking, swelling, and converting between sol and gel forms. In this embodiment, the pathogen detection reagent is incorporated into the smart hydrogel while it is swollen. The smart hydrogel does not release the pathogen detection reagent during sample filtration. When an environmental stimulus is applied to shrink the smart hydrogel after filtration, the pathogen detection reagent is released from the smart hydrogel and disperses throughout the filter (Figure 5). The smart hydrogels or hydrogel particles with pathogen detection agents can be assembled on the top, between, or throughout the filter matrix, as in Figures 4A-4C. In another embodiment, the pathogen detection reagent is incorporated into the smart hydrogel while it is in sol form. The smart hydrogel in gel form does not release the pathogen detection reagent during sample filtration. When an environmental stimulus is applied to convert the smart hydrogel to sol form after filtration, the pathogen detection reagent is released from the smart hydrogel and disperses throughout the filter.

Pathogen detection in a large volume of particulate sample using a composite hydrogel filter containing a pathogen detection reagent comprises the following steps, allowing for fewer steps than the method illustrated in Figure 3:
(1) Filter a large volume of particulate sample that may contain the pathogen to be detected through a composite hydrogel filter, thereby collecting a target pathogen in the filter.
(2) If necessary, wash the filter in order to remove any potential inhibitors of the pathogen detection assay or pathogen growth.
(3) Incubate the filter for a period of time sufficient for pathogen growth such that the pathogen will be detectable.
(4) Detect the grown pathogen using a signaling reagent.

### Degradable Filter Membrane

In certain embodiments, a highly porous filter with pathogen recognition capability is degradable or soluble in solvent to collect the immobilized pathogen efficiently after sample filtration. The degradable filter keeps intact before and during the filtration but is degradable by simple treatment. The filter is made of degradable materials, such as synthetic degradable or erodible polymers or natural degradable or erodible polymers or their copolymers. The synthetic degradable polymers include poly(glycolic acid), poly(lactic acid), polyhydroxybutyrate (PHB), polyhydroxyvalerate (PHV), polycaprolactone, polydioxanone, polyanhydrides, polycyanoacrylates, poly(amino acids), poly(amino acids), poly(ortho ester), polyphosphazenes, poly(propylene fumarate), and their copolymers. The natural polymers include collagen, fibrinogen, fibrin, gelatin, cellulose, chitosan, starch, amylose, alginate, dextran, silk, keratin, elastin, actin, myosin, glycosaminoglycans, carrageenan and their copolymers. The degradation of the filter can occur via hydrolysis, oxidative or enzymatic mechanisms.

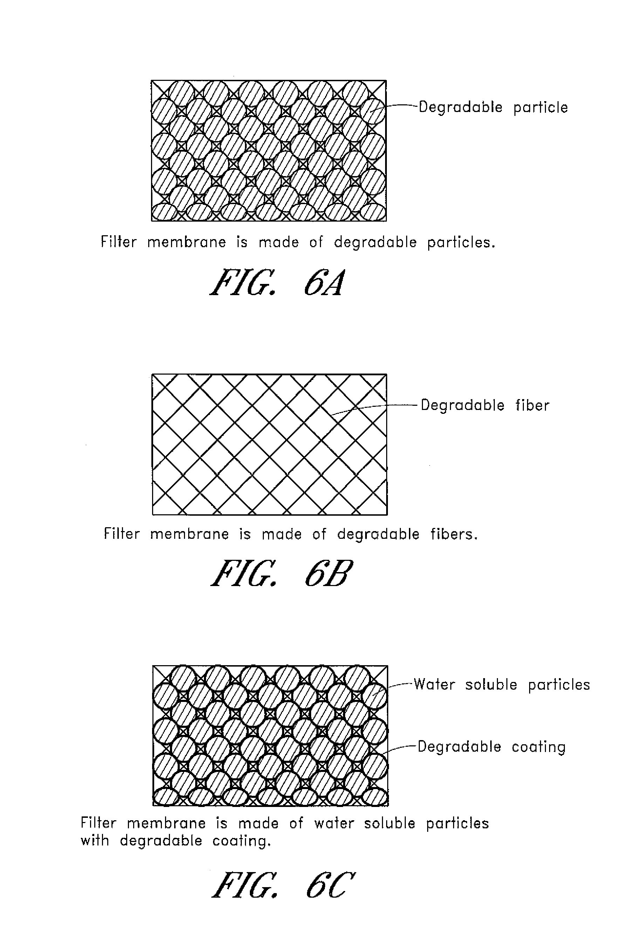

The degradable filter can be fabricated in many ways. In one embodiment, a filter is composed of a degradable polymer so a sample solution can penetrate through pores inside the porous degradable polymer during filtration. In another embodiment, a filter membrane is composed of many degradable polymer particles so the solution can be filtered through the gaps between the degradable polymer particles. Thus the composite degradable filter cannot be blocked during filtration. In another embodiment, a filter is composed of a water-soluble polymer with a degradable polymer coating. In another embodiment, a filter is composed of many water-soluble polymer particles with a degradable polymer coating. Various embodiments of the composite hydrogel filter are illustrated in Figures 6A-6C.

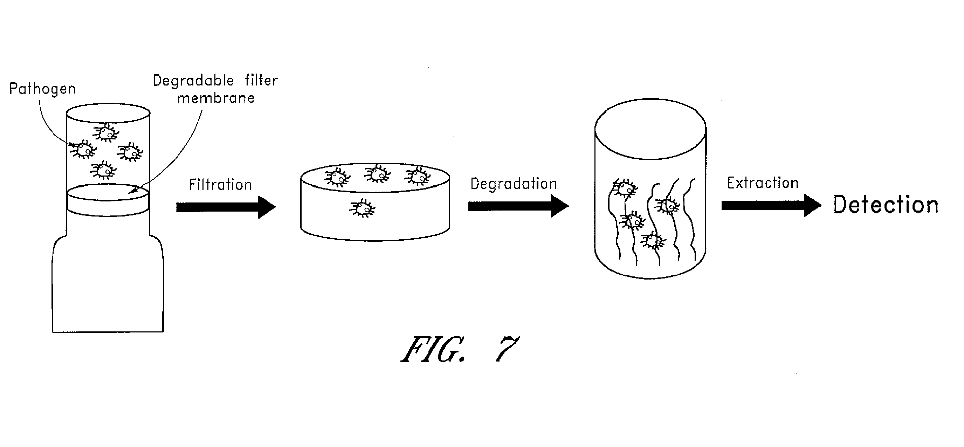

Pathogen detection in a large volume of particulate sample using a degradable filter containing a pathogen detection reagent comprises the following steps (Figure 7):
(1) Filter a large volume of particulate sample that may contain a pathogen to be detected through a degradable filter, thereby collecting the target pathogen in the filter.
(2) Apply a stimulus to make the filter to become a solution.
(3) Collect the pathogen or pathogen-specific molecular marker from the degradation solution.
(4) Detect the pathogen or pathogen-specific molecular marker.

### Example 1: Pathogen Detection Assay in 10% Whole Milk Using Environment-Sensitive Hydrogel

Pathogen detection assays were conducted in 250 mL 10% whole milk inoculated with various doses of *Listeria* cells.

*Listeria innocua* were cultured overnight in BHI broth at 37°C; the *Listeria* concentration was estimated by colony counting after 1/10⁶ dilution. 10% whole milk was prepared by diluting 25 mL whole milk in 225 mL sterile PBS buffer (pH 7.4). The *Listeria* detection reagent was prepared as follows: (1) 11.5 g of Fraser broth base (Oxoid, UK) and 2 g of Bacto agarose were dissolved in 200 mL distilled water, sterilized at 120°C for 20 min, and kept in sol form at 45°C, and (2) the solution was supplemented with 1 vial / 500 mL of Fraser supplement (Oxoid, UK) and 50 mg/L X-glucoside (Sigma, MO).

The pathogen detection assays were conducted as follows: (1) 200 cfu Listeria-inoculated samples or *Listeria* negative samples were filtered with GMF150 1 µm (Whatman, NJ) by vacuum filtration, (2) the GMF150 1 µm filter medium was transferred to a sterile container and kept at 45°C for 5 min, (3) the filter was soaked in 5 mL of *Listeria* detection reagent for 5 min at 45°C and the *Listeria* detection reagent was converted to gel form by cooling to room temperature, and (4) the filter was incubated at 30°C overnight to detect *Listeria* cell growth.

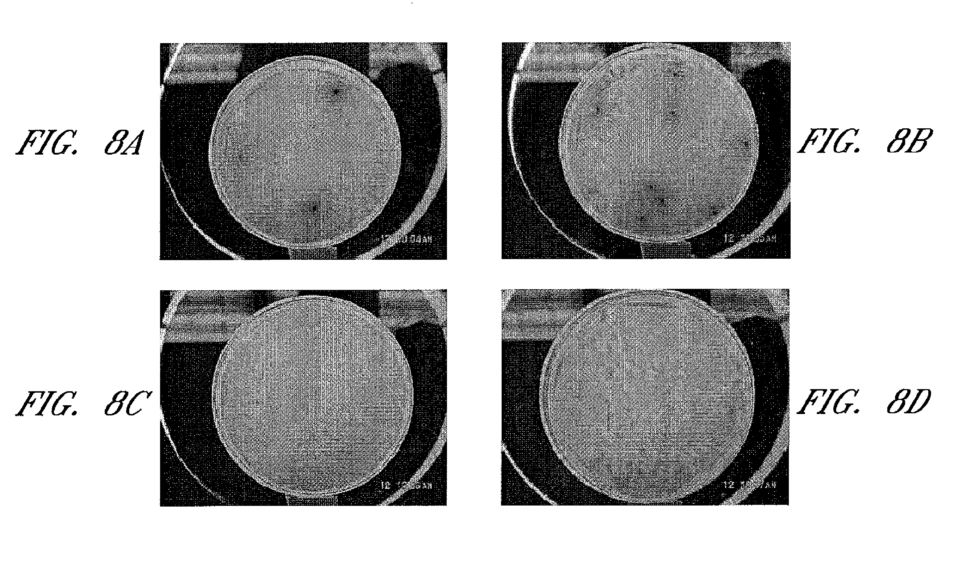

After 24-hour incubation, colony-like colored spots were observed in the filter due to metabolization of X-glucoside by *Listeriα* cells for the *Listeriα-positive* samples. These colorations became much darker at 40 hours than at 24 hours, indicating growth of *Listeria* cells (Figures 8A, 8B). By contrast, *Listeria* negative samples did not show any coloration (Figures 8C, 8D).

### Example 2: Preparation of Hydrogel Particles

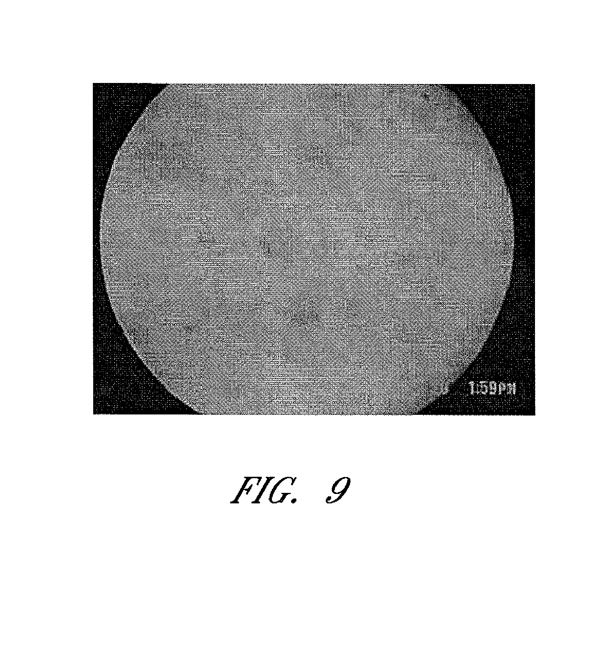

Hydrogel particles were prepared using a water-in-oil (W/O) emulsion method. Low melting point (LMP) agarose (Invitrogen, Carlsbad, CA) solution was dissolved in water at a concentration of 3% (w/v) and kept at 55°C. 1 mL LMP agarose solution was mixed dropwise into 40 mL mineral oil kept at 55°C and stirred vigorously with a magnetic stirrer to form a W/O emulsion. By decreasing the temperature to 4°C while stirring, emulsions containing LMP agarose were gelatinized to form LMP agarose particles. The agarose particles were washed three times with sterile water and kept at 4°C until use. Microscopic analysis confirmed that the agarose particles obtained were spherical in shape and 5 to 50 µm in diameter (Figure 9).

### Example 3: Pathogen Detection Assay Using Hydrogel Microbeads

*Listeria innocua* was freshly cultured in BHI broth at 37°C overnight. Agarose particles (250 µL equivalent) were suspended in 5 mL sterile BHI broth containing 50 mg/L X-glucoside, 8 g/L LiCl₂, and 66.4 µL/mL Rapid L. Mono Supplement 2 (Biorad, CA) for at least one hour at room temperature.

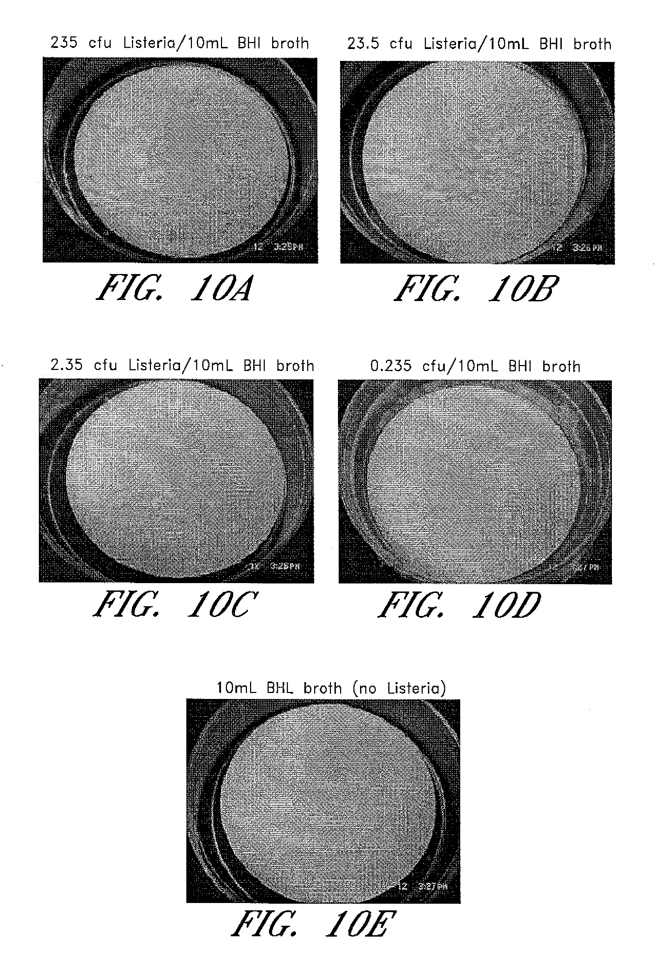

Samples containing a serial dilution of *Listeria* cells were filtered with 47-mm Nanoceram filters (Argonide, FL) using vacuum aspiration. The solution containing suspended agarose particles was applied to the top of the filter, incubated for 1 min, and filtered using vacuum aspiration. The filters containing agarose particles were incubated at 37°C up to two days and blue spots indicative of *Listeria* growth were observed by the naked eye after 17- to 24-hour incubation. The number of blue spots was well correlated with the amount of inoculated *Listeria* cells in the samples (Figure 10).

### Example 4: Filtration of Food Homogenates

The filtration speed of food homogenates was compared on a Nanoceram filter (47-mm diameter, Argonide, FL) or GMF150 1µm (Whatman, NJ) using a 0.5 - 1 g porous spherical microbeads (cross-linked polymethacrylate, EG50OH, Hitachi Chemical, Japan), a 0.5 - 2 g porous microbeads (cross-linked dextran, Sephadex G25, GE Healthcare, NJ), a 15 g non-porous filter aid (glass beads, 40 µm diameter, Filter aid 400, 3M, MN), or no filter aid. Filter aids were deposited on the upstream surface of the Nanoceram filter or GMF150 1 µm filter before filtration of the food homogenates.

Food homogenates were prepared as follows: (1) a 25 g food sample (deli meat, frankfurter or stick cheese) was mixed with 225 mL distilled water, (2) the food sample was stomached manually or by a stomacher (Seward, UK) at 230 rpm for 2 min, and (3) the food homogenate was separated using a filter attached to a stomacher bag (Nasco, WI).

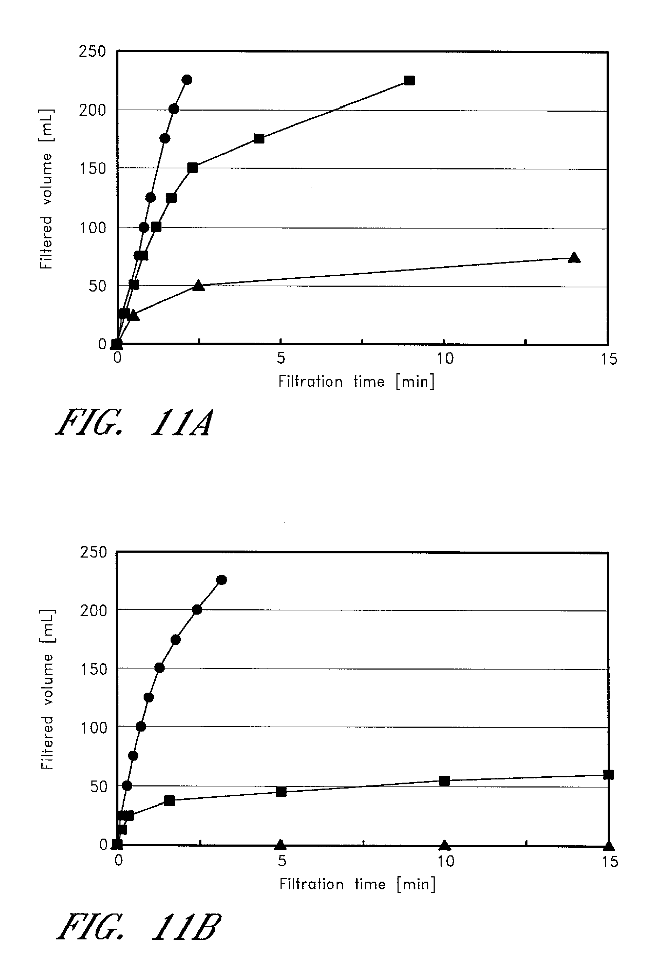

In 10% deli meat homogenate, the Nanoceram filter without filter aid (A) was clogged by less than 100 mL homogenate (Figure 11A). However, using a glass beads (■) or porous microbeads filter aid (cross-linked dextran, 2 g, 86 - 258 µm) (●) allowed filtration of the entire 225 mL homogenates sample without filter clogging. Filtration with the porous microbeads filter aid was completed within 5 min and was much faster than filtration with the glass beads filter aid.

In 10% cheese homogenate, the Nanoceram filters without filter aid (▲) and with a glass beads filter aid (■) were clogged by less than 5 and 75 mL homogenates, respectively (Figure 11B). However, use of a porous microbeads filter aid (cross-linked dextran, 2 g, 86 - 258 µm) (●) allowed the entire 225 mL homogenate to be filtered within 5 min.

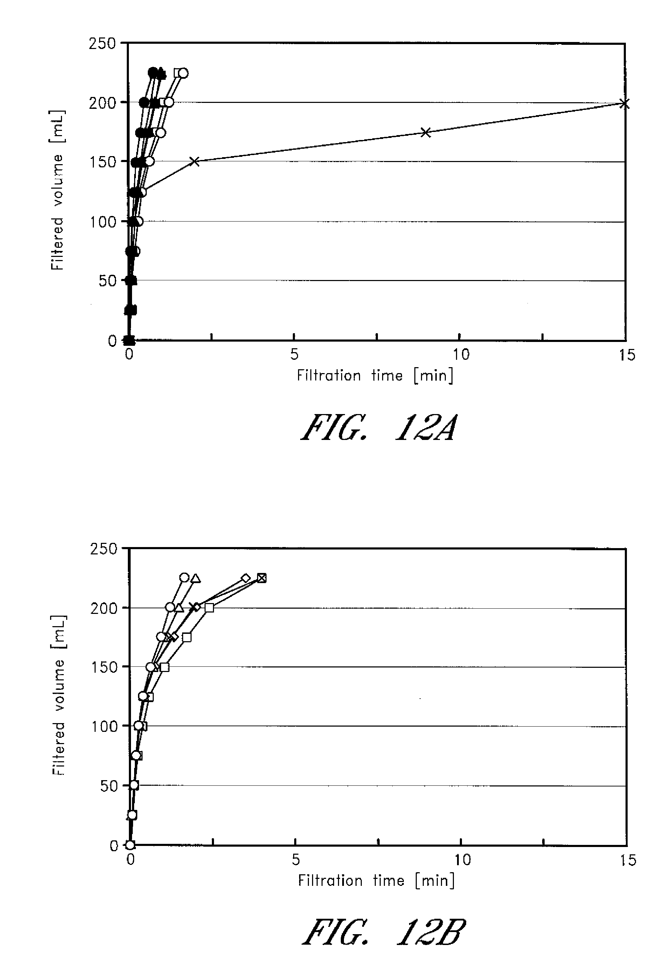

Filtration performance of several grades of porous spherical microbeads filter aids was compared using 10% deli meat homogenates. Cross-linked polymethacrylate microbeads (45 - 90 µm diameter: 0.5 g (○), 1.0 g (●)) and cross-linked dextran microbeads (17 - 70 µm diameter: 0.5 g (□), 35 - 140 µm diameter: 0.5 g (0), 86 - 258 µm diameter: 0.5 g (Δ), 1.0 g (▲), 170 - 520 µm diameter: 0.5 g (×)) were evaluated on GMF150 1µm filters (Figure 12A). The entire 225 mL homogenates were completely filtered within 5 min except cross-linked dextran microbeads with 170 - 520 µm diameter.

Filtration performance of several grades of porous spherical microbeads filter aids was compared using 10% frankfurter homogenates as well. Cross-linked polymethacrylate microbeads (45 - 90 µm diameter: 0.5 g (o)) and cross-linked dextran microbeads (17 - 70 µm diameter: 0.5 g (□), 35 - 140 µm diameter: 0.5 g (0), 86 - 258 µm diameter: 0.5 g (Δ), 170 - 520 µm diameter: 0.5 g (×)) were evaluated on GMF150 1µm filters (Figure 12B). The entire 225 mL homogenates were completely filtered within 5 minutes. These experiments illustrate the superior dirt-holding capacity and filtration capabilities of porous microbeads filter aids over conventional non-porous filter aids.

### Example 5: Pathogen Detection Assay in 225 mL 10% Deli Meat Homogenates

Pathogen detection assays were conducted in 225 mL 10% deli meat homogenates inoculated with various doses of *Listeria* cells.

*Listeria monocytogenes* and *Listeria innocua* were cultured overnight in BHI broth at 37°C; the *Listeria* concentration was estimated by colony counting after 1/10⁶ dilution. 10% deli meat homogenate was prepared as follows: (1) 25 g deli meat was mixed with 225 mL sterile PBS buffer (pH 7.4), (2) the deli meat sample was stomached by a stomacher (Seward, UK) at 230 rpm for 2 min, and (3) the deli meat homogenate was separated using a filter attached to a stomacher bag (Nasco, WI). The deli meat homogenates were inoculated with various doses of *Listeria* cells before filtration. A porous microbeads filter aid (cross-linked dextran, 86 to 258 µm diameter, Sephadex G25, GE Healthcare, NJ) was suspended in BHI broth at 50 g/L and sterilized at 120°C for 20 min. The *Listeria* detection reagent was prepared as follows: (1) 11.5 g of Fraser broth base (Oxoid, UK) and 2 g of Bacto agarose were dissolved in 200 mL distilled water, sterilized at 120°C for 20 min, and kept in sol form at 45°C, and (2) the solution was supplemented with 1 vial / 500 mL of Fraser supplement (Oxoid, UK) and 50 mg/L X-glucoside (Sigma, MO).

The pathogen detection assays were conducted as follows: (1) 0.5 g porous microbeads filter aid was deposited on top of a GMF150 1 µm filter medium (47-mm diameter, Whatman, NJ) and a polyethylene mesh separator (330 µm diameter holes), (2) 0.5 g porous microbeads filter aid was mixed with a *Listeria*-inoculated deli meat homogenate, (3) the deli meat homogenate was filtered by vacuum filtration, (4) the porous microbeads were thoroughly washed three times with 20 mL sterile PBS solution (pH 7.4), (5) the GMF150 1 µm filter medium was transferred to a sterile container and kept at 45°C for 5 min, (6) the filter was soaked in 5 mL of *Listeria* detection reagent for 5 min at 45°C and the *Listeria* detection reagent was converted to gel form by cooling to room temperature, and (7) the filter was incubated at 30°C overnight to detect *Listeria* cell growth.

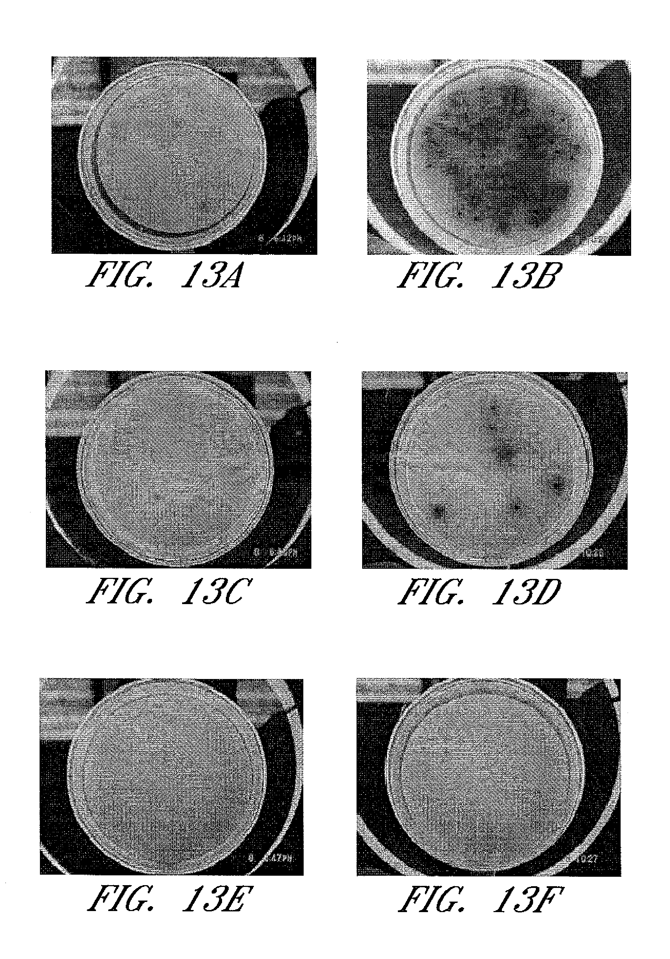

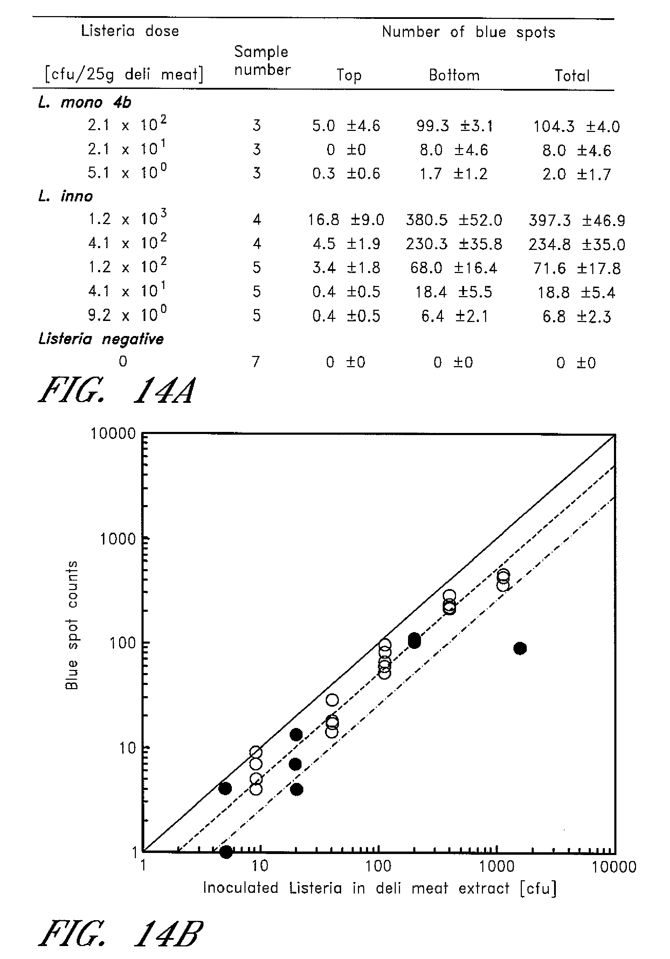

After 24-hour incubation, colony-like colored spots were observed in the filter due to metabolization of X-glucoside by *Listeria* cells (Figures 13A, 13C). These colorations were much darker at 40 hours than at 24 hours, indicating growth of *Listeria* cells (Figures 13B, 13D). By contrast, *Listeriα* negative samples did not show any coloration even after 3-day incubation (Figures 13E, 13F). The number of colored spots was closely correlated to the number of inoculated *Listeria* cells and about 60% of the inoculated *Listeria* cells were collected in the filter medium from ∼225 mL 10% deli meat homogenates (25 g deli meat) (Figures 14A, 14B).

### Example 6: Composite Hydrogel Filter

To fabricate the composite hydrogel filter, a 47-mm Nanoceram filter (Argonide, FL) comprising alumina nanofibers, a pathogen recognition reagent, was used as the matrix to support the hydrogel. Agar hydrogel was used due to its low cost. The new filter was fabricated by filtering the agar gel through the Nanoceram filter. While pathogen recognition reagents, here the alumina nanofibers, ordinarily may become dysfunctional when they are coated with hydrogels on account of a loss of electrostatic interaction with pathogens, here the Nanoceram filter is a depth filter composed of multiple layers, and the top layer was coated with agar hydrogel to prevent this problem from occurring.

The fabrication procedure is discussed below. The top layer of the Nanoceram filter was peeled off the bulk membrane and 1% agar solution was poured on the top layer. The agar solution was then filtered through the top layer under vacuum to maintain the top layer's original porosities. After that, the top layer was air-dried at room temperature and reassembled into the bulk membrane by spraying a small amount of water on it and compressing the layers together using a compressor. The final assembled filter membrane was dried at room temperature.

The fabricated filter membrane was tested using the pathogen solution according to the following procedure:
(1) The filter membrane was washed with 150 ml sterile water.
(2) Approximately 10³ cfu *Listeria* cells in 10 mL BHI broth were applied to the filter and filtered under vacuum.
(3) After filtration, 5 mL BHI broth and 50 mg/L X-glucoside (a chromogenic substrate) were applied to the filter membrane.
(4) The filter membrane was incubated at 37°C for 15 hours.

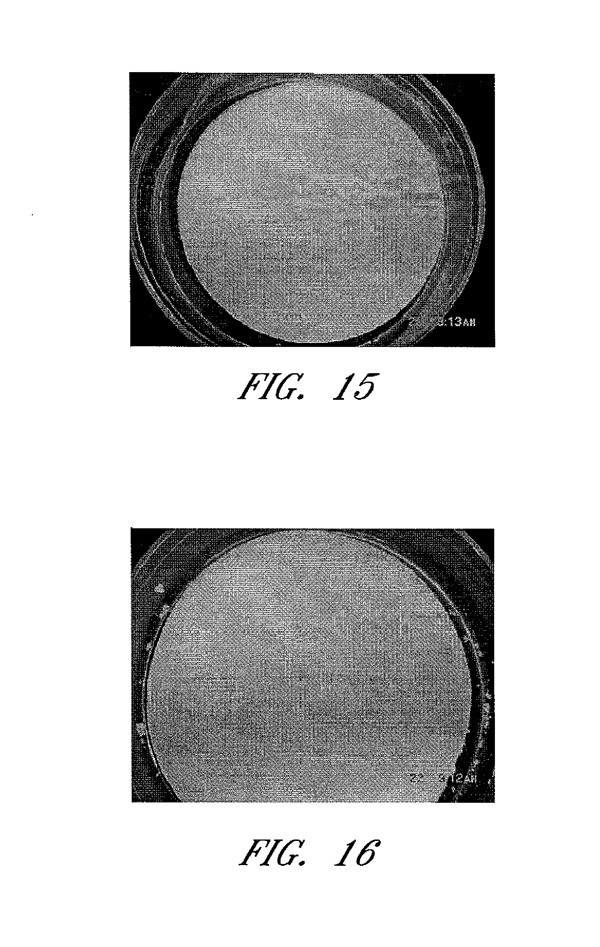

Figure 15 illustrates the result; coloration indicates the existence of Listeria cells that were found on the filter after incubation.

### Example 7: Composite Hydrogel Filter with Pathogen Detection Reagents Immobilized by a Smart Hydrogel

A thermally sensitive hydrogel, poly(N-isopropylacrylamide)/chitosan, was used, as it has the ability to shrink when the temperature reaches 37°C-the incubation temperature. The synthesis comprises the following steps:
(1) Chitosan (0.03 g), acetic acid (240 µl), and NIPAm (1.6 g) were dissolved in distilled water (20 mL).
(2) Crosslinker N,N'-methylenebisacrylamide (0.06 g), initiator 2,2-dimethoxy-2-phenylacetophenone (0.01 g), and catalyst N,N,N',N'-tetramethylethylenediamine (200 µl) were added to the solution and degassed under nitrogen for 40 min.
(3) The solution was photopolymerized in a Petri dish under 365 nm UV-light for two hours.
(4) The hydrogel was soaked in distilled water for three days, changing the water daily.
The smart hydrogel that was generated was ground into small particles and soaked in BHI broth with 200 mg/L X-glucoside (a chromogenic substrate) and heated/cooled several times between 37°C and 25°C. The particles were then collected and assembled using a hydrogel composite Nanoceram filter (as shown in Example 6) by adding the smart hydrogel particles between the top layer and bulk layer as illustrated in Figure 5.

The dried filter membrane was tested using a pathogen solution according to the following procedure:
(1) The filter membrane was washed with 150 mL sterile water.
(2) 5 mL of ∼10⁴ cfu/mL *Listeria* cells in BHI broth were filtered.
(3) The filter was incubated at 37°C for 15 hours in a sealed container.

Figure 16 shows the result; coloration was found on the filter without adding any chromogenic substrate solution after filtration of the sample to be detected.

### Example 8: Degradable Filter Membrane

A degradable filter was fabricated using kappa carrageenan and sodium alginate. 0.4 g of sodium alginate, 0.2 g of kappa carrageenan, and 0.2 g of Pluronic F 127 (surfactant) were dissolved in 30 ml of distilled water at 90°C and then 100 µl of acetic acid was added to the solution. 0.2 g of sodium bicarbonate was transferred into the solution to generate the air bubbles. After the foamy solution was formed, 10 mL of the solution was poured in diameter 60 mm Petri dish. When the solution in Petri dish was cooled down at room temperature, the porous gel was formed. Then the gel membrane was immersed in 0.5M CaCl2 and 0.5M KCl solution for 1 hour to form the crosslinked hydrogel structure. After that, the crosslinked hydrogel structure was freeze-dried under vacuum to make the degradable hydrogel filter.

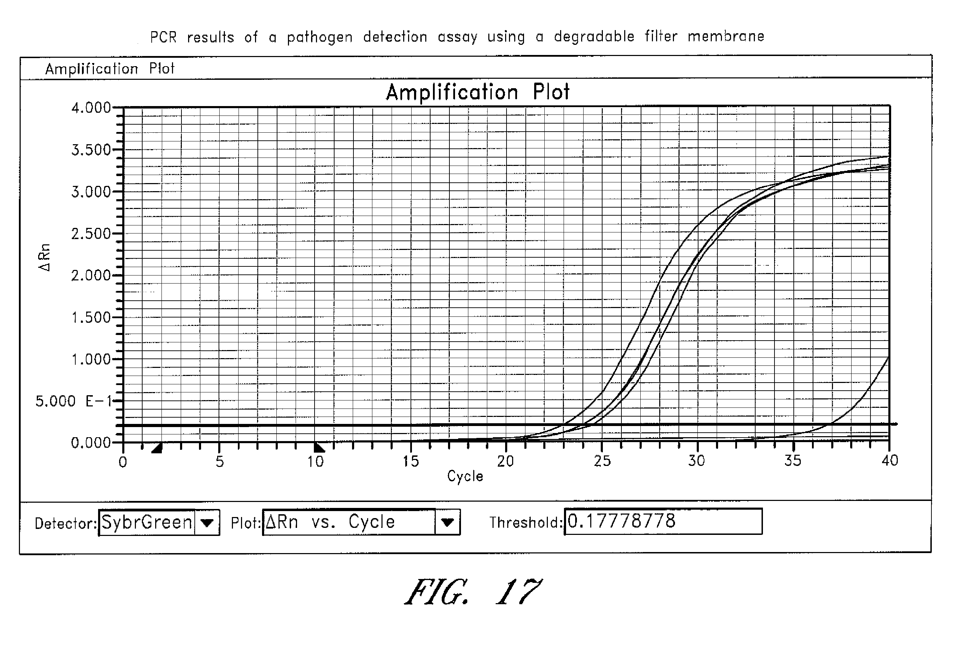

We conducted a pathogen detection assay using the degradable filter. 50 mL of PBS buffer inoculated with ∼109 cfu Listeria innocua was filtered through the degradable hydrogel filter on vacuum aspiration. Then the filter was incubated in 10 mL of 100 mM EDTA solution for 15 minutes at 80oC to make the hydrogel filter dissolved completely. Listeria genomic DNA was extracted from 240 µL of the filter-dissolved solution using a DNA Clean & Concentrator-5 kit (Zymo research, CA) following the instruction manual. The extracted Listeria genomic DNA was detected by real-time PCR on ABI7900 (Applied biosystems, CA) with cycle at threshold (Ct) values of 23 to 24.5 (Figure 17). On the other hand, Listeria genomic DNA was not detected in the negative control, 10 mL of Listeria negative PBS buffer.

It will be understood by those of skill in the art that numerous and various modifications can be made without departing from the spirit of the present invention. Therefore, it should be clearly understood that the forms of the present invention are illustrative only and are not intended to limit the scope of the present invention.

The following numbered clauses, describing aspects of our proposals, are part of the description:
1. A method of detecting a microorganism in a sample comprising:
   filtering the sample through a filter configured to attract the microorganism and having pores configured to prevent clogging during filtration, whereby the microorganism is collected in the filter,
   applying a polymer and a microorganism detection reagent to the filter, wherein the polymer is configured to localize growth of the microorganism and/or prevent evaporation of the microorganism detection reagent;
   incubating the filter for a period of time sufficient to grow the microorganism to detectable level; and
   detecting the presence of the microorganism in the sample.
2. A method of separating a microorganism from a particulate sample comprising filtering the particulate sample with a layered filter composite comprising:
   a filter configured to attract a microorganism and having pores configured to prevent clogging during filtration; and
   a layer of porous spherical microbeads.
3. The method of Clause 2, further comprising detecting said microorganism immobilized in said filter.
4. The method of Clause 1 or 2, further comprising:
   washing the filter after the filtering step for a period of time sufficient to remove substances that inhibit the detection or growth of the microorganism.
5. The method of Clause 1 or 2, wherein the filter is a depth filter comprising a fibrous material.
6. The method of Clause 5, wherein the fibrous material is selected from the group consisting of cellulose fibers and glass microfibers.
7. The method of Clause 1 or 2, wherein the filter is configured to attract the microorganism by an interaction selected from the group consisting of electrostatic, hydrophilic, hydrophobic, physical, and biological interactions.
8. The method of Clause 7, wherein an agent recognizing the microorganism is immobilized specifically or non-specifically on the filter matrix, wherein said agent is selected from the group consisting of an antibody, an antigen, an aptamer, a protein, a nucleic acid, and a carbohydrate.
9. The method of Clause 1, wherein the polymer is a hydrogel.
10. The method of Clause 9, wherein the hydrogel is an environment sensitive hydrogel, and wherein the environment sensitive hydrogel is at least partially in sol form and has low viscosity before application of an environmental change and becomes at least partially gel form with increased viscosity upon application of the environmental change.
11. The method of Clause 10, wherein the environmental change is selected from the group consisting of a change in temperature, pH, amount of incident light, ion concentration, pressure, magnetic field, electric field, sonic radiation, and biochemical molecule concentration.
12. The method of Clause 10, wherein application of the environment sensitive hydrogel to the filter comprises:
   applying a solution comprising at least partially sol form of the environment sensitive hydrogel to the filter;
   gelling the solution by application of the environmental change.
13. The method of Clause 9, wherein the hydrogel is a hydrogel particle.
14. The method of Clause 13, further comprising applying the hydrogel particle by a method selected from the group consisting of absorption, aspiration, filtration, soak and spray of a suspension comprising the hydrogel particle.
15. The method of Clause 13, wherein the hydrogel particle is smaller than the filter pores, thereby occupying the filter pores efficiently.
16. The method of Clause 9, wherein the hydrogel is applied to the filter pores in dehydrated form, and then hydrated inside the filter pores.
17. The method of Clause 16, wherein hydration of the dehydrated hydrogel takes a period of time from 1 minute to 6 hours, wherein dehydrated hydrogel is applied to the filter pores in a solution, and is hydrated inside the pores by incubation for said period of time.
18. The method of Clause 1, wherein the polymer is a viscous polymer solution, and wherein the viscous polymer solution flows into the filter, thereby filling the pores and localizing the growth of the microorganism effectively.
19. The method of Clause 18, the viscous polymer solution comprises a water-soluble polymer or hydrogel suspension.
20. The method of Clause 1, wherein the filter further comprises a layer of porous spherical microbeads.
21. The method of Clause 2 or 20, wherein the layer of porous spherical microbeads is placed on the upstream surface of the filter.
22. The method of Clause 2 or 20, wherein the layer of porous spherical microbeads is placed on the upstream surface of a mesh support and wherein said mesh support is placed on the filter or upstream of the filter.
23. The method of Clause 2 or 20, wherein said layer of porous spherical microbeads forms a homogeneous or graded layer.
24. The method of Clause 2 or 20, wherein the surface of said layer of porous spherical microbeads is highly inert and has low non-specific binding to the microorganism.
25. The method of Clause 2 or 20, wherein the diameter of the microbeads is between 1 and 1000 µm.
26. The method of Clause 2 or 20, wherein the diameter of the microbeads is between 15 and 600 µm.
27. The method of Clause 2 or 20, wherein the diameter of the microbeads is between 50 and 300 µm.
28. The method of Clause 2 or 20, wherein the pore size of the microbeads is smaller than the size of the microorganism, whereby the microorganism passes among the microbeads during the filtration step.
29. The method of Clause 2 or 20, wherein the microbeads comprise a cross-linked hydrophilic polymer.
30. The method of Clause 2 or 20, further comprising:
   disrupting the layer of porous spherical microbeads by adding a wash medium after the filtration step;
   suspending said microbeads; and
   filtering the wash medium with the filter.
31. The method of Clause 1, wherein the microorganism detection reagent contains a growth medium configured to selectively promote the growth of the microorganism.
32. The method of Clause 31, wherein the growth medium comprises a selective reagent or antibiotic to which the microorganism is resistant but other organisms are not resistant, a selected pH and temperature for fast growth of the microorganism and/or slow or inhibited growth of other organisms, or a nutrient readily metabolized by the microorganism but not readily metabolized by other organisms.
33. The method of Clause 31, wherein the growth medium comprises sufficient oxygen for the growth of aerobic bacteria or insufficient oxygen for the growth of anaerobic bacteria.
34. The method of Clause 31, wherein the detecting step comprises detecting a signal from a signaling reagent configured to generate the signal in the presence of the microorganism, and thereby detecting the microorganism.
35. The method of Clause 34, wherein the signaling reagent is selected from the group consisting of a substrate for an enzyme specific to the microorganism, a chromogenic or fluorogenic substrate for an enzyme specific to the microorganism, and a combination of a substrate for an enzyme specific to the microorganism and a chromogenic or fluorogenic indicator to detect digestion of the substrate.
36. The method of Clause 34, wherein the signaling reagent is an agent recognizing the microorganism or a cellular component of the microorganism, selected from the group consisting of an antibody, an antigen, an atpamer, a protein, a nucleic acid and a carbohydrate, wherein the agent comprises a label selected from a dye molecule, a metal colloid, a polymer particle, a magnetic particle, a semiconductive particle, a liposome, a polymersome, a protein, an enzyme and a nucleic acid.
37. A kit for detecting a microorganism comprising:
   a filter configured to attract the microorganism and having pores configured to prevent clogging during filtration;
   a microorganism detection reagent configured to detect the presence of the microorganism; and
   a polymer configured to localize growth of the microorganism and/or prevent evaporation of the microorganism detection reagent.
38. The kit of Clause 37, wherein the polymer is a hydrogel.
39. The kit of Clause 37, wherein the filter is a depth filter comprising a fibrous material.
40. The kit of Clause 39, wherein the fibrous material is selected from the group consisting of cellulose fibers and glass microfibers.
41. The kit of Clause 37, wherein the filter is configured to attract the microorganism by an interaction selected from the group consisting of electrostatic, hydrophilic, hydrophobic, physical, and biological interactions.
42. The kit of Clause 37, wherein the detection reagent recognizing the microorganism is immobilized specifically or non-specifically on the filter matrix, and wherein said agent is selected from the group consisting of an antibody, an antigen, an aptamer, a protein, a nucleic acid, and a carbohydrate.
43. The kit of Clause 3 8, wherein the hydrogel is an environment sensitive hydrogel, and wherein the environment sensitive hydrogel is at least partially in sol form and has low viscosity before application of an environmental change and becomes at least partially gel form with increased viscosity upon application of the environmental change.
44. The kit of Clause 43, wherein the environmental change is selected from the group consisting of a change in temperature, pH, amount of incident light, ion concentration, pressure, magnetic field, electric field, sonic radiation, and biochemical molecule concentration.
45. The kit of Clause 38, wherein the hydrogel is a hydrogel particle.
46. The kit of Clause 45, wherein the hydrogel particle is smaller than the filter pores, thereby occupying the filter pores efficiently.
47. The kit of Clause 38, wherein the hydrogel is in dehydrated form.
48. The kit of Clause 37, wherein the filter further comprises a layer of porous spherical microbeads.
49. The kit of Clause 48, wherein the layer of porous spherical microbeads is placed on the upstream surface of the filter.
50. The kit of Clause 48, wherein the layer of porous Clause cal microbeads is placed on the upstream surface of a mesh support and wherein said mesh support is placed on the filter or upstream of the filter.
51. The kit of Clause 48, wherein said layer of porous spherical microbeads forms a homogeneous or graded layer.
52. The kit of Clause 48, wherein the surface of said layer of porous spherical microbeads is highly inert and has low non-specific binding to the microorganism.
53. The kit of Clause 48, wherein the diameter of the microbeads is between 1 and 1000 µm.
54. The kit of Clause 48, wherein the diameter of the microbeads is between 15 and 600 µm.
55. The kit of Clause 48, wherein the diameter of the microbeads is between 50 and 300 µm.
56. The kit of Clause 48, wherein the pore size of the microbeads is smaller than the size of the microorganism, whereby the microorganism passes among the microbeads during the filtration step.
57. The kit of Clause 48, wherein the microbeads comprise a cross-linked hydrophilic polymer.
58. The kit of Clause 37, wherein the microorganism detection reagent contains a growth medium configured to selectively promote the growth of the microorganism.
59. The kit of Clause 58, wherein the growth medium comprises a selective reagent or antibiotic to which the microorganism is resistant but other organisms are not resistant, a selected pH and temperature for fast growth of the microorganism and/or slow or inhibited growth of other organisms, or a nutrient readily metabolized by the microorganism but not readily metabolized by other organisms.
60. The kit of Clause 58, wherein the growth medium comprises sufficient oxygen for the growth of aerobic bacteria or insufficient oxygen for the growth of anaerobic bacteria.
61. The kit of Clause 58, wherein the growth medium comprises a signaling reagent configured to generate the signal in the presence of the microorganism, and thereby detecting the microorganism.
62. The kit of Clause 61, wherein the signaling reagent is selected from the group consisting of a substrate for an enzyme specific to the microorganism, a chromogenic or fluorogenic substrate for an enzyme specific to the microorganism, and a combination of a substrate for an enzyme specific to the microorganism and an chromogenic or fluorogenic indicator to detect digestion of the substrate.
63. The kit of Clause 61, wherein the signaling reagent is an agent recognizing the microorganism or a cellular component of the microorganism, selected from the group consisting of an antibody, an antigen, an atpamer, a protein, a nucleic acid and a carbohydrate, wherein the agent comprises a label selected from a dye molecule, a metal colloid, a polymer particle, a magnetic particle, a semiconductive particle, a liposome, a polymersome, a protein, an enzyme and a nucleic acid.
64. A layered filter composite for detecting a microorganism comprising:
   a filter medium configured to attract a microorganism and having pores configured to prevent clogging during filtration; and
   a layer of porous spherical microbeads.
65. The layered filter composite of Clause 64, wherein the filter medium is a depth filter comprising a fibrous material.
66. The layered filter composite of Clause 64, wherein the fibrous material is selected from the group consisting of cellulose fibers and glass microfibers.
67. The layered filter composite of Clause 64, wherein the filter medium is configured to attract the microorganism by an interaction selected from the group consisting of electrostatic, hydrophilic, hydrophobic, physical, and biological interactions.
68. The layered filter composite of Clause 67, wherein an agent recognizing the microorganism is immobilized specifically or non-specifically on the filter matrix, wherein said agent is selected from the group consisting of an antibody, an antigen, an aptamer, a protein, a nucleic acid, and a carbohydrate.
69. The layered filter composite of Clause 64, wherein the layer of porous spherical microbeads is placed on the upstream surface of the filter.
70. The layered filter composite of Clause 64, wherein the layer of porous spherical microbeads is placed on the upstream surface of a mesh support and wherein said mesh support is placed on the filter or upstream of the filter.
71. The layered filter composite of Clause 64, wherein said layer of porous spherical microbeads forms a homogenous or graded layer.
72. The layered filter composite of Clause 64, wherein the surface of said layer of porous spherical microbeads is highly inert and has low non-specific binding to the microorganism.
73. The layered filter composite of Clause 64, wherein the diameter of the microbeads is between 1 and 1000 µm.
74. The layered filter composite of Clause 64, wherein the diameter of the microbeads is between 15 and 600 µm.
75. The layered filter composite of Clause 64, wherein the diameter of the microbeads is between 50 and 300 µm.
76. The layered filter composite of Clause 64, wherein the pore size of the microbeads is smaller than the size of the microorganism, whereby the microorganism passes among the microbeads during the filtration step.
77. The layered filter composite of Clause 64, wherein the microbeads comprise a cross-linked hydrophilic polymer.
78. A porous filter, comprising:
   a hydrogel which is a three-dimensional polymer network that can swell, but does not dissolve in water; and
   a filter configured to attract a microorganism and having pores configured to prevent clogging during filtration.
79. The porous filter according to Clause 78, wherein the hydrogel is in dehydrated form.
80. The porous filter according to Clause 78, wherein the filter comprises a filter matrix comprising fibrous materials.
81. The porous filter according to Clause 78, wherein the three-dimensional polymer network comprises a polymer selected from the group consisting of agar, chitosan, cellulose, carrageenan, alginate, acrylate polymer and copolymer, polyethylene glycol, poly(glycolic acid), poly(lactic acid), pectin, locust bean gum, polyhydroxybutyrate (PHB), polyhydroxyvalerate (PHV), polycaprolactone, polydioxanone, polyanhydrides, polycyanoacrylates, poly(amino acids), poly(ortho ester), polyphosphazenes, poly(propylene fumarate), poly(alkylene oxalates), silicone polymers, collagen, fibrinogen, fibrin, gelatin, starch, amylose, dextran, silk, keratin, elastin, actin, myosin, glycosaminoglycans, and polyvinyl alcohol.
82. The porous filter according to Clause 78, wherein the filter is configured to attract the microorganism by an interaction selected from the group consisting of electrostatic, hydrophilic, hydrophobic, physical, and biological interactions.
83. The porous filter according to Clause 82, wherein an agent recognizing the microorganism is immobilized specifically or non-specifically on the filter matrix, wherein said agent is selected from the group consisting of a metal hydroxide or metal oxide, an antibody, an antigen, an aptamer, a protein, a nucleic acid, and a carbohydrate.
84. The porous filter according to Clause 83, wherein the metal hydroxide or metal oxide is selected from the group consisting of zirconium hydroxide, titanium hydroxide, hafnium oxide, hydroxyapatite, iron oxide, titanium oxide, and aluminum oxide.
85. The porous filter according to Clause 78, wherein the filter contains a microorganism detection reagent, and wherein said filter retains said microorganism detection reagent during filtration of a microorganism containing sample.
86. The porous filter of Clause 85, wherein said microorganism detection reagent comprises a growth medium configured to selectively promote the growth of the microorganism to be detected and a signaling reagent configured to generate a signal in the presence of the microorganism.
87. The porous filter of Clause 86, wherein the growth medium comprises a selective reagent or antibiotic to which the microorganism is resistant but other organisms are not resistant, a selected pH and temperature for fast growth of the microorganism and/or slow or inhibited growth of other organisms, or a nutrient readily metabolized by the microorganism but not readily metabolized by other organisms.
88. The porous filter of Clause 86, wherein the growth medium comprises sufficient oxygen for the growth of aerobic bacteria or insufficient oxygen for the growth of anaerobic bacteria.
89. The porous filter of Clause 86, wherein the signaling reagent is selected from the group consisting of a substrate for an enzyme specific to the microorganism, a chromogenic or fluorogenic substrate for an enzyme specific to the microorganism, and a combination of a substrate for an enzyme specific to the microorganism and a chromogenic or fluorogenic indicator to detect digestion of the substrate.
90. The porous filter of Clause 86, wherein the signaling reagent is an agent recognizing the microorganism or a cellular component of the microorganism, selected from the group consisting of an antibody, an antigen, an atpamer, a protein, a nucleic acid and a carbohydrate, wherein the agent comprises a label selected from a dye molecule, a metal colloid, a polymer particle, a magnetic particle, a semiconductive particle, a liposome, a polymersome, a protein, an enzyme and a nucleic acid.
91. A method of detecting a microorganism in a sample, comprising
   immobilizing a microorganism by filtration of the sample through the porous filter according to Clause 78;
   incubating the filter for a period of time sufficient to grow the microorganism to detectable levels; and
   detecting the microorganism using a signaling reagent.
92. The porous filter of Clause 78, wherein the filter is soluble after filtration.
93. The porous filter of Clause 92, wherein the filter comprises a degradable polymer.
94. The porous filter of Clause 93, wherein said degradable polymer is provided in a form selected from fiber, surface coating, beads, particles, sheet and layer.
95. The porous filter of Clause 93, wherein a said degradable polymer becomes a solution via hydrolysis, oxidative, or enzymatic mechanism.
96. A method of detecting a microorganism in a sample using a degradable porous filter according to Clause 92 comprising
   (a) immobilizing a microorganism by filtration of the sample through the filter;
   (b) degrading the degradable filter to become a solution;
   (c) extracting the microorganism or a molecular marker specific to the microorganism from the solution; and
   (d) detecting the microorganism or the microorganism specific molecular marker, thereby confirming the presence of the microorganism in the sample.

## Claims

1. A method of detecting a microorganism in a sample comprising:
filtering the sample through a filter which is a depth filter comprising a fibrous material configured to attract a microorganism by an interaction selected from the group consisting of electrostatic, hydrophilic, hydrophobic, physical, and biological interactions and having pores configured to prevent clogging during filtration, whereby the microorganism is collected in the filter,
applying a polymer and a microorganism detection reagent to the filter, wherein the polymer is configured to localize growth of the microorganism and/or prevent evaporation of the microorganism detection reagent;
incubating the filter for a period of time sufficient to grow the microorganism to detectable level; and
detecting the presence of the microorganism in the sample.

2. The method of Claim 1, further comprising:
washing the filter after the filtering step for a period of time sufficient to remove substances that inhibit the detection or growth of the microorganism.

3. The method of Claim 1, wherein an agent recognizing the microorganism is immobilized specifically or non-specifically on the filter matrix, wherein said agent is selected from the group consisting of an antibody, an antigen, an aptamer, a protein, a nucleic acid, and a carbohydrate.

4. The method of Claim 1, wherein the polymer is an environment sensitive hydrogel, and wherein the environment sensitive hydrogel is at least partially in sol form and has low viscosity before application of an environmental change and becomes at least partially gel form with increased viscosity upon application of the environmental change.

5. The method of Claim 4, wherein the environmental change is selected from the group consisting of a change in temperature, pH, amount of incident light, ion concentration, pressure, magnetic field, electric field, sonic radiation, and biochemical molecule concentration.

6. The method of Claim 4, wherein application of the environment sensitive hydrogel to the filter comprises:
applying a solution comprising at least partially sol form of the environment sensitive hydrogel to the filter;
gelling the solution by application of the environmental change.

7. The method of Claim 1, wherein the polymer is a hydrogel particle smaller than the filter pores, further comprising applying the hydrogel particle by a method selected from the group consisting of absorption, aspiration, filtration, soak and spray of a suspension comprising the hydrogel particle.

8. The method of Claim 1, wherein the polymer is a viscous polymer solution, comprising a water-soluble polymer or hydrogel suspension, and wherein the viscous polymer solution flows into the filter, thereby filling the pores and localizing the growth of the microorganism effectively.

9. The method of Claim 1, wherein the filter further comprises a layer of porous spherical microbeads on the upstream surface of the filter, wherein the pore size of the microbeads is smaller than the size of the microorganism, whereby the microorganism passes among the microbeads during the filtration step.

10. The method of Claim 9, wherein the surface of said layer of porous spherical microbeads is highly inert and has low non-specific binding to the microorganism.

11. The method of Claim 9, wherein the diameter of the microbeads is between 1 and 1000 µm., preferably between 15 and 600 µm, more preferably between 50 and 300 µm.

12. The method of Claim 9, wherein the microbeads comprise a cross-linked hydrophilic polymer.

13. The method of Claim 9, further comprising:
disrupting the layer of porous spherical microbeads by adding a wash medium after the filtration step;
suspending said microbeads; and
filtering the wash medium with the filter.

14. The method of Claim 1, wherein the microorganism detection reagent contains a growth medium configured to selectively promote the growth of the microorganism.

15. The method of Claim 14, wherein the growth medium comprises a selective reagent or antibiotic to which the microorganism is resistant but other organisms are not resistant, a selected pH and temperature for fast growth of the microorganism and/or slow or inhibited growth of other organisms, or a nutrient readily metabolized by the microorganism but not readily metabolized by other organisms.

16. The method of Claim 14, wherein the growth medium comprises sufficient oxygen for the growth of aerobic bacteria or insufficient oxygen for the growth of anaerobic bacteria.

17. The method of Claim 14, wherein the detecting step comprises detecting a signal from a signaling reagent configured to generate the signal in the presence of the microorganism, and thereby detecting the microorganism.

18. The method of Claim 17, wherein the signaling reagent is selected from the group consisting of a substrate for an enzyme specific to the microorganism, a chromogenic or fluorogenic substrate for an enzyme specific to the microorganism, and a combination of a substrate for an enzyme specific to the microorganism and a chromogenic or fluorogenic indicator to detect digestion of the substrate.

19. The method of Claim 17, wherein the signaling reagent is an agent recognizing the microorganism or a cellular component of the microorganism, selected from the group consisting of an antibody, an antigen, an atpamer, a protein, a nucleic acid and a carbohydrate, wherein the agent comprises a label selected from a dye molecule, a metal colloid, a polymer particle, a magnetic particle, a semiconductive particle, a liposome, a polymersome, a protein, an enzyme and a nucleic acid.
